(19) Europäisches Patentamt
European Patent Office
Office européen des brevets

(11) **EP 3 776 068 B1**

(12) **EUROPÄISCHE PATENTSCHRIFT**

(45) Veröffentlichungstag und Bekanntmachung des Hinweises auf die Patenterteilung:
**27.09.2023 Patentblatt 2023/39**

(21) Anmeldenummer: **19715434.7**

(22) Anmeldetag: **28.03.2019**

(51) Internationale Patentklassifikation (IPC):
**G02C 7/02** $^{(2006.01)}$

(52) Gemeinsame Patentklassifikation (CPC):
**G02C 7/027**; G02C 2202/22

(86) Internationale Anmeldenummer:
**PCT/EP2019/057820**

(87) Internationale Veröffentlichungsnummer:
**WO 2019/185770 (03.10.2019 Gazette 2019/40)**

(54) **ANGLEICH DER SUBJEKTIVEN UND OBJEKTIVEN REFRAKTIONEN**

ADJUSTMENT OF SUBJECTIVE AND OBJECTIVE REFRACTIONS

AJUSTEMENT DES RÉFRACTIONS SUBJECTIVE ET OBJECTIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.03.2018 DE 102018002630**

(43) Veröffentlichungstag der Anmeldung:
**17.02.2021 Patentblatt 2021/07**

(60) Teilanmeldung:
**21185761.0 / 3 926 390**

(73) Patentinhaber: **Rodenstock GmbH**
**80687 München (DE)**

(72) Erfinder:
• **MUSCHIELOK, Adam**
**81476 München (DE)**
• **ALTHEIMER, Helmut**
**87650 Baisweil-Lauchdorf (DE)**
• **BECKEN, Wolfgang**
**82061 Neuried (DE)**
• **ESSER, Gregor**
**80686 München (DE)**
• **UTTENWEILER, Dietmar**
**82057 Icking (DE)**

(74) Vertreter: **Müller-Boré & Partner**
**Patentanwälte PartG mbB**
**Friedenheimer Brücke 21**
**80639 München (DE)**

(56) Entgegenhaltungen:
US-A1- 2011 255 053     US-A1- 2012 069 297
US-A1- 2013 100 410     US-A1- 2014 218 681

EP 3 776 068 B1

**Beschreibung**

Die vorliegende Erfindung betrifft Verfahren zum Bestimmen der Fehlsichtigkeit eines Brillenträgers, entsprechende Computerprogrammprodukte, Brillenglasherstellungsverfahren und Vorrichtungen. Ferner betrifft die vorliegende Erfindung ein Brillenglas oder eine Brillenglasserie.

Eine weitverbreitete Methode zum Bestimmen einer Refraktion (umfassend zumindest eine Refraktionskomponente) ist die sogenannte subjektive Refraktionsbestimmung, die sich bei Optikern allgemein durchgesetzt hat. Bei der subjektiven Refraktionsbestimmung werden herkömmlicherweise dem Benutzer eines Brillenglases unterschiedliche Refraktionsgläser vorgesetzt, wobei der Benutzer des Brillenglases dem Refraktionisten eine Verbesserung bzw. eine Verschlechterung des Seheindrucks bei Änderung der optischen Eigenschaften des vorgesetzten Refraktionsglases mitteilt. Die subjektive Refraktion erfordert somit eine Auskunft des Untersuchten über den Seheindruck und kann auch den Einfluss weiterer Größen auf den Seheindruck berücksichtigen.

Die subjektive Refraktionsbestimmung kann beispielsweise auf Werten einer objektiven Refraktionsbestimmung oder auf Werten einer bereits getragenen Brille aufbauen. Die Genauigkeit der subjektiven Refraktionsbestimmung hängt jedoch kritisch vom Können des Refraktionisten, beispielsweise eines Augenoptikers und/oder eines Augenarztes ab, der die subjektive Refraktionsbestimmung durchführt. Ebenso hängt die subjektive Refraktionsbestimmung kritisch von der zu untersuchenden Person ab, insbesondere von der Fähigkeit der zu untersuchenden Person die Schärfe der Seheindrücke zu beurteilen und/oder zu artikulieren.

Eine andere Methode zum Bestimmen einer Refraktion ist die so genannte objektive Refraktion: Die objektive Refraktion wird mittels einer apparativen Anordnung durchgeführt und wird durch die Brechungseigenschaften sowie die Geometrie des Augapfels bestimmt. Die objektive Refraktion kann mittels verschiedener Geräte durchgeführt werden, wie z.B. Refraktometer, Häufig unterscheiden sich jedoch die mittels einer objektiven Refraktion ermittelten Werte eines Brillenträgers erheblich von den mittels einer subjektiven Refraktion ermittelten Werten. Dies erschwert erheblich die Auffindung von geeigneten Sollwerten für das Brillenglas, welche Fehlsichtigkeit des Brillenträgers korrigieren sollen.

Die Druckschrift WO 2009/007136 A1 beschreibt ein Verfahren zum Bestimmen von Sollwerten für ein Brillenglas, bei dem zumindest eine Teilmenge der subjektiven Refraktionsdaten an die objektiven Refraktionsdaten aufgrund eines Vergleichs der subjektiven und der objektiven Daten angepasst wird. Insbesondere wird die Teilmenge der subjektiven Refraktionsdaten an die objektiven Refraktionsdaten angepasst, wenn das Vergleichsergebnis zumindest eine vorbestimmte Vergleichsbedingung erfüllt, ansonsten wird die Teilmenge der subjektiven Refraktionsdaten beibehalten.

Die Druckschrift US 2012/069297 beschreibt ein Verfahren zur Bestimmung der Refraktionswerte eines Benutzers, bei dem sowohl Wellenfrontmessungen zumindest eines Auges des Benutzers als auch subjektive Refraktionswerte berücksichtigt werden.

Die Druckschriften US 2014/368795 A1 und US 2014/368795 A1 beschreiben jeweils Verfahren zur Bestimmung der Refraktionswerte eines Benutzers mittels objektiver und subjektiver Refraktion.

Es ist eine Aufgabe der vorliegenden Erfindung, die Bestimmung der Fehlsichtigkeit eines Brillenträgers zu verbessern. Diese Aufgabe wird gelöst anhand der Verfahren, Vorrichtungen, Computerprogrammprodukte, Brillengläser und Brillenglasserien nach den unabhängigen Ansprüchen. Bevorzugte Ausführungsvarianten bzw. -formen sind Gegenstand der abhängigen Ansprüche.

Die vorliegende Erfindung basiert auf der Erkenntnis, dass unterschiedliche Messungen bzw. Messverfahren und/oder Messgeräte grundsätzlich unterschiedliche Refraktionswerte liefern. Die Erfindung schlägt vor, bei der Berechnung der Fehlsichtigkeit eines Brillenträgers Messungenauigkeiten bzw. Messabweichungen der unterschiedlichen Messungen zu berücksichtigen.

Gemäß einem ersten Aspekt der Erfindung wird ein im Anspruch 1 definiertes, computerimplementiertes bzw. computergestütztes Verfahren zum Bestimmen der Fehlsichtigkeit eines Auges eines Brillenträgers beschrieben, wobei das Verfahren umfasst:

Bereitstellen von Messwerten aus einer ersten und einer zweiten Messung der Fehlsichtigkeit des Auges des Brillenträgers;

Berechnen eines Schätzwerts für die Fehlsichtigkeit des Auges des Brillenträgers anhand der Messwerte aus der ersten und der zweiten Messung, wobei bei der Berechnung des Schätzwerts der Fehlsichtigkeit Messungenauigkeiten bzw. Messabweichungen der ersten und der zweiten Messungen der Fehlsichtigkeit berücksichtigt werden bzw. in die Berechnung des Schätzwerts der Fehlsichtigkeit Messungenauigkeiten bzw. Messabweichungen der ersten Messung und der zweiten Messung der Fehlsichtigkeit einfließen.

"Bereitstellen" im Sinne der vorliegenden Erfindung beinhaltet "Entnehmen einer Datenbank, einer Tabelle oder einem anderen Datenträger", "Eingeben in einer Benutzerschnittstelle, wie z.B. einer graphischen Benutzerschnittstelle", "Übermitteln", "Messen" oder "Schätzen".

Die erste und die zweite Messung können mittels Messvorrichtungen zur Messung der Fehlsichtigkeit verschiedener Art, z.B. mittels verschiedener Geräte, subjektiv/objektiv, etc., erfolgen.

Die Messwerte können gemessene Werte zumindest einer Komponente, vorzugsweise mehrerer Komponenten umfassen. Anders ausgedrückt können die Messwerte in vektorieller Form mit mehreren Komponenten vorliegen. Die Komponenten können z.B.

> die Komponenten einer Polardarstellung (Sphäre, Zylinder und Achse),
> die Komponenten einer Krümmungsmatrix-Darstellung,
> die Komponenten einer Powervektor-Darstellung (M, J0 und J45),
> die Komponenten einer Harris-Vektor-Darstellung,
> die Komponenten einer Zernike-Polynom Zerlegung (Zernike-Koeffizienten), oder
> die Komponente einer anderen geeigneten Charakterisierung der Fehlsichtigkeit eines Brillenträgers sein.

Die Messungenauigkeiten bzw. Messabweichungen der ersten und der zweiten Messungen können vorab bestimmt (z.B. gemäß eines der unten beschriebenen Verfahren) und in einer geeigneten Form gespeichert werden (z.B. als Tabelle, in einer Datei, in einer Datenbank, als ein mathematisches Modell, als eine Funktion, etc.). Das Verfahren kann dementsprechend Bereitstellen von Daten bzw. Informationen über die Messungenauigkeiten bzw. Messabweichungen der ersten und der zweiten Messungen der Fehlsichtigkeit umfassen. Ferner kann das Verfahren Bereitstellen von Daten über die Art der Messung, das jeweils verwendete Gerät, individuellen Daten des Brillenträgers (wie z.B. Alter, Präferenzen, Sehgewohnheiten, Verwendung des Brillenglases, Parameter der Gebrauchsstellung des Brillenglases, etc.) umfassen.

Gemäß einem zweiten Aspekt wird ein Verfahren zum Bestimmen der Sollwirkung eines Brillenglases zur Korrektur einer Fehlsichtigkeit eines Brillenträgers vorgeschlagen, wobei das Verfahren umfasst:

> Bestimmen der Fehlsichtigkeit eines Auges des Brillenträgers nach dem Verfahren gemäß dem ersten Aspekt; und
> Festlegen der Sollwirkung anhand der bestimmten Fehlsichtigkeit, so dass die Sollwirkung die bestimmte Fehlsichtigkeit in zumindest einem Bezugspunkt zumindest teilweise, vorzugsweise im Wesentlichen vollständig, korrigiert.

Der Bezugspunkt kann der Fernbezugspunkt, der Prismenbezugspunkt, der Zentrierpunkt bzw. das Zentrierkreuz, der Nahbezugspunkt oder ein anderer geeigneter Bezugspunkt sein.

Gemäß einem dritten Aspekt wird ein Verfahren zum Herstellen eines Brillenglases vorgeschlagen, wobei das Verfahren umfasst:

> Bestimmen der Fehlsichtigkeit eines Auges des Brillenträgers nach dem Verfahren gemäß dem zweiten Aspekt;
> Festlegen der Sollwirkung in zumindest einem Bezugspunkt des Brillenglases anhand der bestimmten Fehlsichtigkeit, so dass die Sollwirkung des Brillenglases die bestimmte Fehlsichtigkeit in dem zumindest einen Bezugspunkt zumindest teilweise, vorzugsweise im Wesentlichen vollständig, korrigiert; und
> Fertigen des Brillenglases, so dass die Sollwirkung in dem zumindest einen Bezugspunkt des Brillenglases, vorzugsweise in einer vorgegebenen Gebrauchsstellung des Brillenglases, erreicht wird.

Gemäß einem vierten Aspekt wird ein Verfahren zum Bestellen von Brillengläsern vorgeschlagen, umfassend:

> Bereitstellen von Messwerten aus einer ersten Messung und einer zweiten Messung der Fehlsichtigkeit des Auges des Brillenträgers;
> Berechnen eines Schätzwerts für die Fehlsichtigkeit des Auges des Brillenträgers anhand der Messwerte aus der ersten Messung und der zweiten Messung, wobei bei der Berechnung des Schätzwerts der Fehlsichtigkeit Messungenauigkeiten bzw. Messabweichungen der ersten Messung und der zweiten Messung der Fehlsichtigkeit berücksichtigt werden.

Gemäß einem fünften Aspekt wird ein Computerprogrammprodukt vorgeschlagen, welches, wenn geladen in den Speicher eines Computers und ausgeführt auf einem Computer, bewirkt, dass der Computer ein Verfahren nach einem der obigen Aspekte durchführt.

Gemäß einem sechsten Aspekt der Erfindung wird eine Vorrichtung zum Bestimmen der Fehlsichtigkeit eines Auges eines Brillenträgers mit einer Rechenvorrichtung, insbesondere ein Computer oder Computersystem, vorgeschlagen, welche ausgebildet ist, das Verfahren nach einem der obigen Aspekte durchzuführen.

Gemäß einem siebten Aspekt der Erfindung wir eine Vorrichtung zum Herstellen eines Brillenglases vorgeschlagen, wobei die Vorrichtung umfasst:

> eine Vorrichtung zum Bestimmen der Fehlsichtigkeit eines Auges eines Brillenträgers gemäß dem sechsten Aspekt;

eine Vorrichtung zum Festlegen der Sollwirkung in einem Bezugspunkt des Brillenglases anhand der bestimmten Fehlsichtigkeit, so dass die Sollwirkung des Brillenglases die bestimmte Fehlsichtigkeit in dem zumindest einen Bezugspunkt zumindest teilweise, vorzugsweise im Wesentlichen vollständig korrigiert; und

eine Fertigungsvorrichtung zum Fertigen des Brillenglases, so dass die Sollwirkung in zumindest einem vorgegebenen Bezugspunkt des Brillenglases, vorzugsweise in einer vorgegebenen Gebrauchsstellung des Brillenglases, erreicht wird.

Gemäß einem achten Aspekt der Erfindung wird eine Vorrichtung zum Bestellen von Brillengläsern vorgeschlagen, die ausgebildet ist, das Verfahren zum Bestellen von Brillengläsern durchzuführen. Insbesondere umfasst die Vorrichtung zum Bestellen von Brillengläsern:

eine Vorrichtung zum Bereitstellen von Messwerten aus einer ersten Messung und einer zweiten Messung der Fehlsichtigkeit des Auges des Brillenträgers, und

eine Rechenvorrichtung welche ausgebildet ist, einen Schätzwert für die Fehlsichtigkeit des Auges des Brillenträgers anhand der Messwerte aus der ersten Messung und der zweiten Messung zu berechnen, wobei in die Berechnung des Schätzwerts der Fehlsichtigkeit Messungenauigkeiten bzw. Messabweichungen der ersten Messung und der zweiten Messung der Fehlsichtigkeit einfließen. Der Schätzwert kann nach einem der oben beschriebenen Verfahren ermittelt werden.

Die oben genannten Vorrichtungen zum Bereitstellen, Bestimmen oder Festlegen oder Berechnen von Daten und/oder Messwerten können durch geeignet konfigurierte bzw. programmierte Datenverarbeitungsvorrichtungen (insbesondere spezialisierte Hardwaremodule, Computer oder Computersysteme) mit entsprechenden Recheneinheiten, elektronische Schnittstellen, Speicher und Datenübermittlungseinheiten realisiert werden. Die Vorrichtungen können ferner zumindest eine vorzugsweise interaktive grafische Benutzerschnittstelle (GUI) umfassen, welche es einem Benutzer ermöglicht, Daten einzugeben und/oder zu modifizieren.

Die Fertigungsvorrichtung kann z.B. zumindest eine CNC gesteuerte Maschine zur Direktbearbeitung eines Blanks nach den ermittelten Optimierungsvorgaben umfassen. Alternativ kann das Brillenglas mittels eines Gießverfahrens gefertigt werden. Vorzugsweise weist das fertig bearbeitete Brillenglas eine einfache sphärische oder rotationssymmetrisch asphärische Fläche und eine nach dem erfindungsgemäßen Verfahren sowie nach individuellen Parametern des Brillenträgers berechnete oder optimierte Fläche auf. Vorzugsweise ist die einfache sphärische oder rotationssymmetrisch asphärische Fläche die Vorderfläche (d.h. die objektseitige Fläche) des Brillenglases. Selbstverständlich ist es jedoch möglich, die optimierte Fläche als Vorderfläche des Brillenglases anzuordnen. Auch können beide Flächen des Brillenglases optimiert werden.

Des Weiteren bietet die Erfindung eine Verwendung eine nach dem Herstellungsverfahren gemäß der Erfindung hergestellten Brillenglases in einer vorgegebenen durchschnittlichen oder idealen Gebrauchsstellung des Brillenglases vor den Augen eines bestimmten Brillenträgers zur Korrektion einer Fehlsichtigkeit des Brillenträgers, wobei die Fehlsichtigkeit durch einen mittels einer ersten Messvorrichtung ermittelten Messwert und einen mittels einer zweiten Messvorrichtung ermittelten Messwert charakterisiert ist.

Die Verfahren, Vorrichtungen und Computerprogrammprodukte gemäß einem der obigen Aspekte können die Reklamationswahrscheinlichkeit von Brillengläsern verkleinern, bei deren Berechnung sowohl die subjektive als auch die objektive Refraktion verwendet wird. Dies bezieht sich speziell auf Wirkungsbereiche, in denen die Geräte zur objektiven Refraktion systematisch anders als die subjektive Refraktion messen.

Bevorzugte Beispiele

Die Messungenauigkeiten umfassen eine systematische Abweichung zwischen den Messwerten aus der ersten Messung von den Messwerten aus der zweiten Messung.

Wird beispielsweise der systematischen Abweichung zwischen der ersten und zweiten Messung keine Rechnung getragen, kann es zu erheblichen Abweichungen in den nach dem Stand der Technik ermittelten, z.B. gemittelten, Refraktionswerten von den für den Brillenträger optimalen Werten kommen.

Gemäß einem bevorzugten Beispiel umfassen die Messungenauigkeiten bzw. Messabweichungen sowohl eine statistische als auch eine systematische Abweichung der ersten Messung von der zweiten Messung. Die systematischen und statistischen Abweichungen können in einem einzigen Verfahrensschritt, oder aber in mehreren Verfahrensschritten nacheinander in einer beliebigen Reihenfolge berücksichtigt werden.

Gemäß der Erfindung wird zunächst ein erster Schätzwert für die Fehlsichtigkeit des Auges des Brillenträgers anhand der ersten und der zweiten Messung berechnet, wobei bei dem Berechnen des ersten Schätzwerts der Fehlsichtigkeit systematische Abweichungen zwischen den Messwerten aus der ersten Messung und der zweiten Messung der Fehlsichtigkeit berücksichtigt werden. In einem zweiten Schritt wird anhand des ersten Schätzwerts und der statistischen Messungenauigkeiten bzw. Messabweichungen der ersten und der zweiten Messungen ein zweiter Schätzwert der Fehlsichtigkeit ermittelt, der als der endgültige Schätzwert ausgegeben wird oder weiter angepasst wird.

Das Bestimmen des ersten Schätzwerts kann ein Ermitteln eines Korrekturterms für die Messwerte aus der ersten und/oder der zweiten Messung und eine Korrektur bzw. Anpassung der Messwerte der ersten oder der zweiten Messung anhand des Korrekturterms (z.B. durch eine Addition der jeweiligen Messwerte mit dem Korrekturterm) umfassen.

Der erste Schätzwert kann weiter korrigiert werden, um den statistischen Abweichungen der ersten Messung von der zweiten Messung Rechnung zu tragen. Dies kann z.B. durch eine Kombination der gegebenenfalls korrigierten Messwerte aus der ersten und der zweiten Messung erfolgen, wie nachfolgend in Detail beschrieben wird.

Die Kombination der Messwerte aus der ersten und der zweiten Messung und die Korrektur bzw. Anpassung der Messwerte aus der ersten und der zweiten Messung können ebenfalls in umgekehrter Reihenfolge erfolgen.

Gemäß einem bevorzugten Beispiel ist die erste Messung der Fehlsichtigkeit des Auges eine objektive Refraktion und/oder die zweite Messung der Fehlsichtigkeit des Auges eine subjektive Refraktion. Die Messwerte umfassen entsprechend Werte zumindest einer Komponente bzw. Refraktionskomponente. Diese zumindest eine Komponente der Messwerte kann eine Komponente einer Wellenfrontdarstellung der Fehlsichtigkeit, deren Linearkombination oder daraus abgeleitete Größen sein. Die zumindest eine Komponente kann z.B.:

die Komponente einer Polardarstellung (Sphäre, Zylinder und Achse),
die Komponente einer Krümmungsmatrix-Darstellung,
die Komponente einer Powervektor-Darstellung (M, J0 und J45),
die Komponente einer Harris-Vektor-Darstellung,
die Komponente einer Zernike-Polynom Zerlegung (Zernike-Koeffizient), oder
die Komponente einer anderen geeigneten Charakterisierung der Fehlsichtigkeit eines Brillenträgers sein.

Das Verfahren kann ferner Bereitstellen von Daten über die Messgenauigkeiten bzw. Messabweichungen der ersten und der zweiten Messung der Fehlsichtigkeit umfassen. Die Daten können in elektronischer Form (z.B. gespeichert in einer Datenbank) oder auf einer Form (z.B. auf Papier) gespeichert werden. Die Daten können in tabellarischer Form vorliegen (z.B. als "Look-up Table" (LUT)) oder als ein mathematisches Modell vorgegeben werden, z.B. als parametrische Funktion mit vorgegebenen Parametern.

Das Verfahren umfasst Bestimmen der Messungenauigkeiten bzw. Messabweichungen der ersten und der zweiten Messung mittels statistischer Analyse der in einem Datensatz (Referenzdatensatz) enthaltenen Daten bzw. Messwerte (Referenzmesswerte) aus früheren Messungen (z.B. früheren ersten und zweiten Messungen bzw. Messungen mit Messvorrichtungen der ersten und der zweiten Art) verschiedener Brillenträger. Der Datensatz (Referenzdatensatz) kann ferner auch andere Messungen umfassen anhand denen die Messungenauigkeiten bzw. Messabweichungen der ersten und der zweiten Messung ermittelt werden.

Die rohen Messwerte können vor der Analyse gefiltert werden, z.B. anhand der folgenden Kriterien:

der Betrag einer Differenz aus einer Addition und einem reziproken Objektabstand bei (subjektiver) Nahrefraktionsmessung (bei positiver Vorzeichenkonvention) ist gleich oder kleiner als ein vorbestimmter Schwellenwert, optional gleich oder kleiner als 0 Dpt, 0,25 Dpt oder 0,5 Dpt;
die Sehstärke des jeweiligen Brillenträgers (dessen Refraktionswerte in dem Datensatz enthalten ist) ist gleich oder größer als ein vorbestimmter Schwellenwert, optional gleich oder größer als 1,25 oder 1,5 oder 1,6;
die Auflösung der bei der subjektiven Refraktion eines Brillenträgers verwendeten Refraktionsgläser ist gleich oder höher als ein vorbestimmter Schwellenwert, optional gleich oder größer als 0,5 Dpt oder 0,25 Dpt oder 0,125 Dpt.

Andere Kriterien sind ebenfalls möglich, wie z.B. Dichte der Daten in einem bestimmten Messwertintervall.

Das Bestimmen der Messungenauigkeiten bzw. der Messabweichungen der ersten und der zweiten Messungen umfasst zum Beispiel die folgenden Schritte:

Festlegen eines Modells für die Messwerte der zweiten Messung als eine Summe eines vorhergesagten Messwerts und einer Zufallsvariable, wobei der vorhergesagte Messwert modelliert ist als eine parametrische Funktion des Messwerts der ersten Messung und optional eines Teils des Messwerts der zweiten Messung;
Ermitteln der Parameter der parametrischen Funktion durch Anpassen des Modells an die im Datensatz enthaltenen Referenzmessungen unter Maximieren der Wahrscheinlichkeitsverteilung der Zufallsvariablen im Parameterraum des Modells;
Bestimmen einer systematischen Abweichung der ersten Messung von der zweiten Messung anhand der vorhergesagten Messung.

Das Modell kann z.B. durch die folgende Gleichung bzw. das folgende Gleichungssystem beschrieben werden:

$$\tilde{P}_2 = P_{pred}[\tilde{P}_1, \dots] + \varepsilon,$$

wobei:

$P_1$ den Messwert der ersten Messung (in vektorieller Form) bezeichnet;
$P_2$ den Messwert der zweiten Messung (in vektorieller Form) bezeichnet;
$P_{pred}$ den vorhergesagten Messwert (in vektorieller Form) bezeichnet; und
$\varepsilon$ die Zufallsvariable (in vektorieller Form) bezeichnet.

Die obige Gleichung bzw. das obige Gleichungssystem ist separat für jeden Messwert im Referenzdatensatz zu betrachten, d.h. gilt für jede Messung "i". Für i-te Messung gilt folglich:

$$\tilde{P^i}_2 = P_{pred}[\tilde{P^i}_1, \dots] + \varepsilon^i$$

Jeder Messung kann folglich eine Zufallsvariable $\varepsilon^i$ (die eine vektorielle Größe sein kann) zugeordnet werden. Alle Zufallsvariablen $\varepsilon^i$ stemmen aus derselben Verteilung bzw. beziehen sich auf derselben Verteilung.

Wenn optional die parametrische Funktion eine Funktion des Messwerts der ersten Messung und eines Teils des Messwerts der zweiten Messung ist, fließt vorzugsweise die Komponente der zweiten Messung, welche modelliert wird, nicht in die parametrische Funktion ein. Ansonsten gibt es eine triviale Lösung, nämlich dass die Zufallsvariable immer 0 beträgt, und die parametrische Funktion ist identisch mit der zu modellierenden Komponente der zweiten Messung.

Die vorhergesagte Messung kann durch eine beliebige parametrische Funktion modelliert werden, z.B. durch eine polynomische Funktion. Die vorhergesagte Messung kann zum Beispiel eine vorhergesagte Refraktion sein, welche durch eine der folgenden parametrischen Funktionen modelliert werden kann:

Modell 1:

$$M_{pred}\left(\tilde{M}_{obj}, \tilde{J0}_{obj}, \widetilde{J45}_{obj}\right) = \sum_{i=0}^{4} a_{M,i}^{M} \tilde{M}_{obj}^{i} + a_{J0,1}^{M} \tilde{J0}_{obj} + a_{J45,1}^{M} \widetilde{J45}_{obj}$$

$$J0_{pred}\left(\tilde{M}_{obj}, \tilde{J0}_{obj}, \widetilde{J45}_{obj}\right) = a_{M,1}^{J0} \tilde{M}_{obj} + \sum_{i=0}^{4} a_{J0,i}^{J0} \tilde{J0}_{obj}^{i} + a_{J45,1}^{J0} \widetilde{J45}_{obj}$$

$$J45_{pred}\left(\tilde{M}_{obj}, \tilde{J0}_{obj}, \widetilde{J45}_{obj}\right) = a_{M,1}^{J45} \tilde{M}_{obj} + a_{J0,1}^{J45} \widetilde{J45}_{obj} + \sum_{i=0}^{4} a_{J45,i}^{J45} \widetilde{J45}_{obj}^{i}$$

oder

Modell 2:

$$M_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj} + b_{J0,1}^{M} \widetilde{J0}_{sub} + b_{J45,1}^{M} \widetilde{J45}_{sub}$$

$$J0_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj} + b_{M,1}^{J0} \widetilde{M}_{sub} + b_{J45,1}^{J0} \widetilde{J45}_{sub}$$

$$J45_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i} + b_{M,1}^{J45} \widetilde{M}_{sub} + b_{J0,1}^{J45} \widetilde{J0}_{sub}$$

wobei:

$(M_{pred}, J0_{pred}, J45_{pred})$ den Powervektor der vorhergesagten Refraktion bezeichnet;

$(\widetilde{M}_{obj}, \widetilde{J0}_{obj}, \widetilde{J45}_{obj})$ den Powervektor der Messwerte aus der objektiven Refraktion bezeichnet;

$(\widetilde{M}_{sub}, \widetilde{J0}_{sub}, \widetilde{J45}_{sub})$ den Powervektor der Messwerte aus der subjektiven Refraktion bezeichnet;

$a_{X,i}^{Y}$ die Parameter der jeweiligen parametrischen Funktion bezeichnen,
$Y$ für eine Powervektor-Komponente des Powervektors der vorhergesagten Refraktion steht;
$X$ für eine Powervektor-Komponente des Powervektors der gemessenen objektiven Refraktion steht.

Die ermittelten Parameter $a_{X,i}^{Y}$ können in geeigneter Form (z.B. als LUT) gespeichert werden und bei dem Berechnen des Schätzwerts für die Fehlsichtigkeit berücksichtigt werden.

Anhand der vorhergesagten Messung gemäß dem Modell und der bereitgestellten subjektiven und objektiven Messwerte kann die systematische Abweichung der ersten Messung von der zweiten Messung und entsprechende Korrekturterme bestimmt werden. Dabei kann der objektive Messwert, der subjektive Messwert oder beide Messwerte korrigiert werden (z.B. durch Addition des jeweiligen Messwerts mit dem bestimmten Korrekturterm).

Ferner wird vorgeschlagen, die statistischen Messfehler bzw. Messungenauigkeiten durch eine Kombination der Messwerte aus der ersten Messung und der zweiten Messung (z.B. die subjektiven und die objektiven Refraktionswerte) zu minimieren. Als besonders vorteilhaft hat sich erwiesen, den Schätzwert der Fehlsichtigkeit des Auges durch Bilden eines gewichteten Mittelwertes der Messwerte aus der ersten und der zweiten Messung zu berechnen, wobei die erste Messung bzw. die Komponenten des Messwerts aus der ersten Messung mit ersten Gewichten und die zweite Messung bzw. die Komponenten des Messwert aus der zweiten Messung mit zweiten Gewichten gewichtet wird und die Summe des ersten und des zweiten Gewichts für die jeweilige Komponente gleich 1 ist. Da es sich bei den Messwerten im Prinzip um vektorielle Größen (d.h. um Größen mit mehreren Komponenten) handelt, werden die einzelnen Komponenten (z.-B. Powervektor-Komponenten) im Allgemeinen mit unterschiedlichen Gewichten gewichtet. Wenn der jeweilige Messwert lediglich eine Komponente aufweist (z.B. das sphärische Äquivalent, wird die Komponente aus der ersten Messung mit einem ersten Gewicht und die Komponente aus der zweiten Messung mit einem zweiten Gewicht gewichtet.

Vorzugsweise wird unter der ersten Messung und der zweiten Messung diejenige Messung mit der geringeren Messungenauigkeit mit höheren Gewichten gewichtet. Vorzugsweise werden zuvor die Messewerte aus der ersten Messung und/oder die Messwerte aus der zweiten Messung korrigiert bzw. abgeändert, um die statistischen Abweichungen zwischen der ersten und der zweiten Messung zu reduzieren.

Die Gewichte sind vorzugsweise abhängig von den Messwerten der Fehlsichtigkeit. Die Messwerte können zum Beispiel eine Addition und/oder ein sphärisches Äquivalent umfassen und die Gewichte können abhängig von der Addition und/oder der Differenz zwischen dem Messwert des sphärischen Äquivalents aus der ersten Messung und dem Messwert des sphärischen Äquivalents aus der zweiten Messung sein. Gemäß einem Aspekt wird eine neuartige Gewichtung vorgeschlagen, um die statischen Messungenauigkeiten bzw. Abweichungen einer objektiven und einer subjektiven Messung der Fehlsichtigkeit zu minimieren.

Wenn z.B. die Addition gleich oder höher als ein vorbestimmter Wert ist (z.B. 1,75 Dpt oder 2,0 Dpt oder 2,25 Dpt oder 2,5 Dpt) bzw. äquivalent das Akkommodationsvermögen gleich oder niedriger als ein vorbestimmter Wert ist (z.B. 0,75 Dpt oder 0,5 Dpt oder 0,25 Dpt oder 0 Dpt) ist, und wenn die Differenz bzw. der Unterschied $\Delta M$ zwischen dem objektiven sphärischen Äquivalent und dem subjektiven sphärischen Äquivalent nicht groß ist (z.B. im Intervall -0,75 Dpt < $\Delta M$ < +0,75 Dpt oder -0,5 Dpt < $\Delta M$ < +0,5 Dpt liegt) ist das Gewicht des subjektiven sphärischen Äquivalents zwischen 0,3 und 0,7.

Wenn die Addition gleich oder höher als ein vorbestimmter Wert ist (z.B. 1,75 Dpt oder 2,0 Dpt oder 2,25 Dpt oder 2,5 Dpt) bzw. äquivalent das Akkommodationsvermögen gleich oder niedriger als ein vorbestimmter Wert ist (z.B. 0,75 Dpt oder 0,5 Dpt oder 0,25 Dpt oder 0 Dpt), und wenn der Betrag der Differenz zwischen dem objektiven sphärischen Äquivalent und dem subjektiven sphärischen Äquivalent groß ist (z.B. größer als 1,5 Dpt oder 1,0 Dpt oder 0,5 Dpt) ist das Gewicht des subjektiven sphärischen Äquivalents größer oder gleich 0,8 oder 0,9 oder 0,95 oder 0,99. Der Wert kann sogar 1 sein.

Wenn die Addition gleich oder niedriger als ein vorbestimmter Wert ist (z.B. gleich oder kleiner als 1,5 Dpt oder 1,25 Dpt oder 1,0 Dpt oder 0,75 Dpt oder 0,5 Dpt) bzw. äquivalent das Akkommodationsvermögen gleich oder größer als ein vorbestimmter Wert ist (z.B. gleich oder höher als 1,0 Dpt oder 1,25 Dpt oder 1,5 Dpt oder 1,75 Dpt oder 2,0 Dpt), und wenn die Differenz $\Delta M$ zwischen dem objektiven sphärischen Äquivalent und dem subjektiven sphärischen Äquivalent negativ und kleiner als ein vorbestimmter Wert ist, (z.B. kleiner als -0,5 Dpt oder -1,0 Dpt oder -1,5 Dpt), ist das Gewicht des objektiven sphärischen Äquivalents klein, z.B. 0,5 oder 0,4 oder 0,3 oder 0,2 oder 0,1 oder 0,05 oder 0,01. Der Wert kann sogar 0 sein.

Wenn die Addition niedriger als ein vorbestimmter Wert ist, und die Differenz $\Delta M$ zwischen dem objektiven sphärischen Äquivalent und dem subjektiven sphärischen Äquivalent nicht groß ist (z.B. im Intervall -0,75 Dpt < $\Delta M$ < +0,75 Dpt oder -0,5 Dpt < $\Delta M$ < +0,5 Dpt liegt), werden das subjektive und das objektive sphärische Äquivalent ähnlich gewichtet wie beim Vorliegen einer (relativ) hohen Presbyopie. Das Gewicht des subjektiven sphärischen Äquivalents kann z.B. zwischen 0,3 und 0,7 oder zwischen 0,4 und 0,6 sein.

Die Gewichte können ferner auch von anderen Komponenten der Refraktion (Refraktionskomponenten) abhängen, wie z.B. die Komponente J0 und J45 in der Powervektordarstellung.

Die Messungen der Fehlsichtigkeit können ferner zumindest eine astigmatische Komponente umfassen (z.B. die Powervektorkomponenten J0 und J45), wobei die subjektiven und objektiven astigmatischen Komponenten mit konstanten Gewichten gewichtet werden können. Beispielsweise kann das Gewicht für die subjektive astigmatische Komponente 0,7 betragen und das Gewicht für die entsprechende objektive astigmatische Komponente 0,3. Andere Werte sind auch möglich und können ausdrücken, dass beide Messungen dieselbe statistische Ungenauigkeit besitzen (beide Gewichte 0,5) oder dass die subjektiv ermittelten astigmatischen Komponenten eine geringere statistische Ungenauigkeit besitzen (z.B. Gewicht objektiv: 0,7, Gewicht subjektiv: 0,3).

Vorzugsweise werden die Messungenauigkeiten bzw. Messabweichungen der ersten und der zweiten Messung für den gleichen Objektabstand, wie z.B. Objektabstand Unendlich bestimmt bzw. quantifiziert. Ferner werden die Messungenauigkeiten bzw. Messabweichungen vorzugsweise bei einer für alle Daten identischen Entfernung zum Auge bestimmt bzw. quantifiziert. Das Verfahren kann entsprechend Umrechnen von rohen objektiven und/oder subjektiven Refraktionswerten auf eine gemeinsame Entfernung zum Auge bzw. auf eine gemeinsame Ebene oder Fläche umfassen, wobei die Entfernung beispielsweise die Entfernung vom Hornhautscheitel oder von der Eintrittspupille des Auges sein kann.

Weiter bevorzugt werden die Messungenauigkeiten bzw. die Messabweichungen der ersten und der zweiten Messung separat für verschiedene Geräte zum Bestimmen von objektiven Refraktionswerten bestimmt bzw. quantifiziert.

Das obige Verfahren kann mittels einer entsprechend ausgebildeten Vorrichtung durchgeführt werden. Die Vorrichtung kann eine Rechen- bzw. Datenverarbeitungsvorrichtung (insbesondere ein Computer oder Computersystem) umfassen, welche programmiert ist, das Verfahren und insbesondere das Berechnen des Schätzwerts durchzuführen. Ferner kann die Vorrichtung geeignete Schnittstellen aufweisen, die eine Übermittlung oder Eingabe oder Auslesen von Messwerten aus einer ersten und einer zweiten Messung ermöglichen. Ebenfalls kann die Vorrichtung eine Speichereinheit umfassen, welche die Messwerte aus der ersten und der zweiten Messung und gegebenenfalls zuvor ermittelte Messungenauigkeiten bzw. Messabweichungen (z.B. in tabellarischer Form oder in Form eines Modells) speichert.

Die Vorrichtung zum Bestimmen der Fehlsichtigkeit eines Auges eines Brillenträgers kann ferner zumindest eine Messvorrichtung einer ersten Art zum Durchführen der ersten Messung, insbesondere eine Messvorrichtung zum Durchrühren einer objektiven Refraktionsmessung umfassen. Vorzugsweise ist die Rechenvorrichtung ausgebildet, wie oben beschrieben, die systematischen Abweichungen der mit der Messvorrichtung zum Durchführen einer objektiven Refraktionsmessung (objektive Messvorrichtung) enthaltenen Messwerte von den mit einer subjektiven Messung enthaltenen Messwerte zumindest teilweise zu kompensieren.

Die Vorrichtung kann ferner eine zweite Messvorrichtung einer zweiten Art zum Durchführen der zweiten Messung, insbesondere eine Messvorrichtung zum Durchführen einer subjektiven Refraktionsmessung umfassen.

Das Verfahren zum Bestimmen der Fehlsichtigkeit eines Brillenträgers kann Bestandteil eines Verfahrens zum Bestellen und/oder Herstellen eines Brillenglases sein. Dementsprechend kann die Vorrichtung zum Bestimmen der Fehlsichtigkeit eines Brillenträgers Bestandteil einer Vorrichtung zum Bestellen und/oder Herstellen eines Brillenglases sein. Das Verfahren zum Bestellen und/oder Herstellen eines Brillenglases kann ferner Festlegen einer Sollwirkung des Brillenglases anhand der bestimmten Fehlsichtigkeit umfassen. Die Sollwirkung des Brillenglases wird so festgelegt, dass die bestimmte Fehlsichtigkeit in zumindest einem Bezugspunkt des Brillenglases (wie z.B. im Fernbezugspunkt oder im Prismenbezugspunkt oder im Zentrierkreuz und optional im Nahbezugspunkt) zumindest teilweise, vorzugsweise im Wesentlichen korrigiert wird. Das Verfahren kann ferner Berechnen und Fertigen des Brillenglases umfassen, wobei das Brillenglas so berechnet und gefertigt wird, dass dessen Wirkung in dem zumindest einem Bezugspunkt wesentlich gleich der Sollwirkung ist. Vorzugsweise erfolgt die Berechnung in einer individuell für den Brillenträger vorbestimmten oder einer durchschnittlichen Gebrauchsstellung. Die Gebrauchsstellung kann durch Parameter wie Hornhautscheitelabstand, Augendrehpunktabstand, Vorneigung, Fassungsscheibenwinkel, Pupillenabstand, Pupillendurchmesser, etc. charakterisiert werden.

Ein weiterer Aspekt der Erfindung betrifft ein Brillenglas oder ein Satz aus einem Brillenglas und einer Spezifikation der Fehlsichtigkeit, welche das Brillenglas korrigieren soll, wobei das Brillenglas nach dem obigen Verfahren hergestellt werden kann. Ebenfalls wird eine Serie von Brillengläsern oder von Sätzen von Brillengläsern mit zugeordneten Spezifikationen der Fehlsichtigkeit vorgeschlagen. Die Spezifikation der Fehlsichtigkeit, die durch ein bestimmtes Brillenglas korrigiert werden soll, kann insofern als Bestandteil des Brillenglases angesehen werden.

Das Brillenglas weist eine erste Wirkung $P\_A$ in einem Bezugspunkt des Brillenglases auf. Der Bezugspunkt kann z.B. der Fernbezugspunkt, der Prismenbezugspunkt, das Zentrierkreuz, der Nahbezugspunkt oder ein anderer geeigneter Bezugspunkt sein. Die Wirkung kann, wie oben beschrieben, mehrere Komponenten aufweisen, wie z.B. eine sphärische und/oder eine astigmatische Komponente.

Die Fehlsichtigkeit (welche Bestandteil der Spezifikation für das Brillenglas sein kann) kann durch einen ersten Messwert $P\_A1$ und einen zweiten Messwert $P\_A2$ charakterisiert werden, wobei die Messwerte mehrere Komponenten umfassen können (wie z.B. eine sphärische, eine astigmatische Komponente, etc.). Die Komponenten der Messwerte der Fehlsichtigkeit entsprechen im Allgemeinen den Komponenten der Wirkung im Bezugspunkt des Brillenglases.

Der erste Messwert $P\_A1$ und der zweite Messwert $P\_A2$ werden mittels unterschiedlicher Messungen erhalten. Insbesondere wird der erste Messwert $P\_A1$ mittels einer Messvorrichtung der ersten Art zur Messung der Fehlsichtigkeit erhalten und der zweite Messwert $P\_A2$ mittels einer Messvorrichtung der zweiten Art zur Messung der Fehlsichtigkeit erhalten. In der Regel unterscheiden sich der erste Messwert $P\_A1$ und der zweite Messwert $P\_A2$ in zumindest einer Komponente X.

Die Komponente X der im Bezugspunkt des Brillenglases vorhandenen Wirkung $P\_A$ liegt näher an der Komponente X desjenigen Messwerts unter den Messwerten $P\_A1$ oder $P\_A2$, welcher von der Messvorrichtung mit der geringeren Ungenauigkeit bei der Messung der Komponente X erhalten wird. Wie oben dargelegt, können die Komponenten der Messwerte $P\_A1$ und $P\_A2$ Komponenten einer Wellenfrontdarstellung der Fehlsichtigkeit, deren Linearkombination oder daraus abgeleitete Größen sein. Vorzugsweise sind die Komponente X der im Bezugspunkt des ersten Brillenglases vorhandenen Wirkung $P\_A$ und die Komponente X des ersten Messwerts des ersten Auges $P\_A1$ im Wesentlichen identisch.

Das Brillenglas kann ein Einstärkenbrillenglas (mit oder ohne astigmatischer Wirkung) oder ein progressives Brillenglas sein.

Die obigen Brillengläser können eine Serie von Brillengläsern mit unterschiedlichen Wirkungen in dem zumindest einen Bezugspunkt bilden, wobei die Brillengläser unterschiedliche Fehlsichtigkeiten korrigieren. Eine solche Serie kann zum Beispiel zumindest drei Brillengläser mit unterschiedlichen Wirkungen im Bezugspunkt umfassen:

ein erstes Brillenglas A zur Korrektur einer ersten Fehlsichtigkeit,
ein zweites Brillenglas B zur Korrektur einer zweiten Fehlsichtigkeit; und
ein drittes Brillenglas C zur Korrektur einer dritten Fehlsichtigkeit.

Die erste, zweite und dritte Fehlsichtigkeit können jeweils durch zwei unterschiedliche Messwerte charakterisiert werden, wobei die zwei Messwerte mittels unterschiedlicher Messvorrichtungen zur Messung der Fehlsichtigkeit erhalten werden. Die Messvorrichtung bzw. Messvorrichtungen der ersten Art (erste Messvorrichtung(en)) kann bzw. können eine Messvorrichtung bzw. Messvorrichtungen zur Messung der subjektiven Refraktion sein. Die Messvorrichtung bzw. Messvorrichtungen der zweiten Art (zweite Messvorrichtung(en)) kann bzw. können eine Messvorrichtung bzw. Messvorrichtungen zur Messung der objektiven Refraktion sein. Jeder Messwert kann mehrere Komponenten umfassen (z.B. eine sphärische, eine astigmatische Komponente, etc.). Die mit der bzw. den ersten Messvorrichtung(en) erhaltenen Messwerte unterscheiden sich von den mit der bzw. den zweiten Messvorrichtung(en) erhaltenen Messwerten in zumindest einer Komponente.

In dem zumindest einen Bezugspunkt weist das Brillenglas A eine erste Wirkung P_A, das Brillenglas B eine zweite Wirkung P_B und das Brillenglas C eine dritte Wirkung P_C auf. Die erste Fehlsichtigkeit wird durch einen ersten Messwert P_A1 und einen zweiten Messwert P_A2 charakterisiert. Die zweite Fehlsichtigkeit wird durch einen ersten Messwert P_B1 und einen zweiten Messwert P_B2 charakterisiert. Die dritte Fehlsichtigkeit wird durch einen ersten Messwert P_C1 und einen zweiten Messwert P_C2 charakterisiert.

Der erste Bezugspunkt kann der Fernbezugspunkt, der Prismenbezugspunkt, der Zentrierpunkt bzw. das Zentrierkreuz, der Nahbezugspunkt oder ein anderer geeigneter Bezugspunkt sein. Der erste Bezugspunkt kann mittels einer permanenten oder nicht permanenten Markierung im oder auf dem Brillenglas markiert bzw. gekennzeichnet sein.

Die mit der bzw. den Messvorrichtung(en) der ersten Art bestimmten ersten Messwerte P_A1, P_B1 und P_C1 sind komponentenweise identisch. Die Komponenten X der mit der bzw. den Messvorrichtung(en) der zweiten Art bestimmten zweiten Messwerte P_A2, P_B2 und P_C2 sind alle paarweise unterscheiden. Die Komponente X der ersten Wirkung P_A und die Komponente X des ersten Messwerts P_A1 sind nahezu identisch. Für die Komponenten X der im Bezugspunkt vorhandenen Wirkung des i-ten Brillenglases, X_i, wobei i = A, B oder C, und für die Komponenten X der zweiten Messwerte der i-ten Augen, X_i2 gelten vorzugsweise folgende Zusammenhänge:

$$(X\_B - X\_A) / (X\_B2 - X\_A2) \text{ ungleich } (X\_C - X\_A) / (X\_C2 - X\_A2);$$

$$\text{abs}(X\_B2 - X\_A2) < \text{abs}(X\_C2 - X\_A2);$$

und

$$\text{signum}(X\_B2 - X\_A2) = \text{signum}(X\_C2 - X\_A2),$$

wobei die Funktion abs(x) den absoluten Wert des Arguments x angibt und die Funktion signum (x) die Vorzeichenfunktion ist, die dem Argument x sein Vorzeichen zuordnet.

Die Brillengläser können Einstärkengläser (Add = 0 Dpt) oder progressive Brillengläser (Gleitsichtgläser) sein (Add ≠ 0 Dpt), wobei alle progressive Brillengläser in der Serie dieselben Additionen aufweisen.

Vorzugsweise gelten für Einstärkengläser und über dieselbe Addition Add verfügende Gleitsichtgläser mit einer Addition Add <= 1,5, optional 1,25 Dpt die folgenden Zusammenhänge für die Komponenten X der im Bezugspunkt vorhandenen Wirkung des i-ten Brillenglases, X_i, und für die Komponenten X der zweiten Messwerte der i-ten Augen, X_i2:

$$(X\_B - X\_A) / (X\_B2 - X\_A2) < (X\_C - X\_A) / (X\_C2 - X\_A2) \text{ falls } X\_B2 - X\_A2 > 0,$$
$$X\_C2 - X\_A2 > 0,$$

und

$$(X\_B - X\_A) / (X\_B2 - X\_A2) > (X\_C - X\_A) / (X\_C2 - X\_A2) \text{ falls } X\_B2 - X\_A2 < 0,$$
$$X\_C2 - X\_A2 < 0.$$

Vorzugsweise gelten für Gleitsichtgläser mit einer Addition Add >= 2 Dpt folgende Zusammenhänge für die Komponenten X der im Bezugspunkt vorhandenen Wirkung des i-ten Brillenglases, X_i, und für die Komponenten X der zweiten Messwerte der i-ten Augen, X_i2:

$$(X\_B - X\_A) / (X\_B2 - X\_A2) > (X\_C - X\_A) / (X\_C2 - X\_A2) \text{ falls } X\_B2 - X\_A2 > 0,$$
$$X\_C2 - X\_A2 > 0,$$

und

$$(X\_B - X\_A) / (X\_B2 - X\_A2) > (X\_C - X\_A) / (X\_C2 - X\_A2) \quad \text{falls } X\_B2 - X\_A2 < 0,$$

$$X\_C2 - X\_A2 < 0.$$

Die Komponente X kann z.B. das sphärische Äquivalent sein.

Die Serie von Brillengläsern kann ein viertes Brillenglas D zur Korrektur einer vierten Fehlsichtigkeit und ein fünftes Brillenglas E zur Korrektur einer fünften Fehlsichtigkeit umfassen. Das Brillenglas D weist im Bezugspunkt eine vierte Wirkung P_D auf. Das Brillenglas. E weist im Bezugspunkt eine fünfte Wirkung P_E auf. Die vierte Fehlsichtigkeit wird durch zumindest einen ersten Messwert P_D1 und einen zweiten Messwert P_D2 charakterisiert.

Die fünfte Fehlsichtigkeit wird durch zumindest einen ersten Messwert P_E1 und einen zweiten Messwert P_E2 charakterisiert.

Die Messwerte P_D1 und P_E1 werden mittels der bzw. den Messvorrichtung(en) der ersten Art zur Messung der Fehlsichtigkeit erhalten. Die Messwerte P_D2 und P_E2 werden mittels der bzw. den Messvorrichtung(en) der zweiten Art zur Messung der Fehlsichtigkeit erhalten.

Die Messwerte P_D1, P_D2, P_E1 und P_E2 bestehen jeweils vorzugsweise aus mehreren Komponenten. Der erste Messwert P_D1 und der zweite Messwert P_D2 können sich in zumindest einer Komponente X unterscheiden. Ebenfalls können sich der erste Messwert P_E1 und der zweite Messwert P_E2 in zumindest einer Komponente X unterscheiden.

Ferner sind vorzugsweise folgende Bedingungen erfüllt:

die Werte P_A1, P_D1 und P_E1 sind komponentenweise identisch:
die Komponenten X der mit den Messvorrichtungen der zweiten Art bestimmten zweiten Messwerte P_A2, P_D2 und P_E2 des ersten, vierten und fünften Auges unterscheiden sich alle paarweise,
die Komponente X der im Bezugspunkt des ersten Brillenglases vorhandenen ersten Wirkung P_A und die Komponente X des ersten Messwerts des ersten Auges, P_A1, sind nahezu identisch, und
für die Komponenten X der im Bezugspunkt vorhandenen Wirkung des i-ten Brillenglases, X_i, und für die Komponenten X der zweiten Messwerte der i-ten Augen, X_i2, gelten die folgenden Zusammenhänge:

$$X\_D2 - X\_A2 > 0,$$

$$X\_E2 - X\_A2 < 0,$$

$$X\_D - X\_A > 0$$

und

$$X\_E - X\_A < 0.$$

Die Serie kann auch weitere Gläser mit unterschiedlicher Wirkung zur Korrektur unterschiedlicher Fehlsichtigkeiten umfassen.

Nachfolgend werden bevorzugte Ausführungsformen der vorliegenden Erfindung anhand begleitender Figuren beispielhaft beschrieben. Einzelelemente der beschriebenen Ausführungsformen sind nicht auf die jeweilige Ausführungsform beschränkt. Vielmehr können Elemente der Ausführungsformen beliebig miteinander kombiniert werden und neue Ausführungsformen dadurch erstellt werden. Es zeigt bzw. zeigen:

**Figur 1** die systematischen Abweichungen objektiver und subjektiver Wellenfronten für zwei verschiedene Aberrometer (Modell 1);
**Figur 2** die systematischen Abweichungen objektiver und subjektiver Wellenfronten für zwei verschiede Aberrometer (Modell 2);
**Figur 3** die Gewichte des subjektiven sphärischen Äquivalents $g_{sub}$ gemäß einem ersten Beispiel;
**Figur 4** die Gewichte des subjektiven sphärischen Äquivalents $g_{sub}$ gemäß einem dritten zweiten Beispiel (Fig. 4A) und einem dritten Beispiel (Fig. 4B);
**Figur 5** die Änderung des nach zwei unterschiedlichen Verfahren erhaltenen Schätzwerts der Fehlsichtigkeit für einen ersten Aberrometer als Differenz der Werte, welche nach den unterschiedlichen Verfahren erhalten werden;

**Figur 6** die Änderung des nach zwei unterschiedlichen Verfahren erhaltenen Schätzwerts der Fehlsichtigkeit für einen zweiten Aberrometer als Differenz der Werte, welche nach den zwei unterschiedlichen Verfahren erhalten werden;

**Figuren 7** bis **10** beispielhafte Brillengläsern;

**Figuren 11** bis **19** die Differenz zwischen einem Schätzwert des sphärischen Äquivalents und einem gemessenen subjektiven sphärischen Äquivalent als Funktion der Differenz zwischen einem gemessenen objektiven sphärischen Äquivalent und einem gemessenen subjektiven sphärischen Äquivalent für unterschiedliche Additionen.

Im Rahmen der vorliegenden Anmeldung wird Bezug auf folgende Fachterminologie genommen:
Die Messung der Fehlsichtigkeit eines Auges umfasst insbesondere eine subjektive Refraktionsbestimmung, eine objektive Refraktionsbestimmung (z.B. mit einem Refraktometer oder Autorefraktometer) oder eine Wellenfrontmessung. Die objektive Refraktionsbestimmung oder die Wellenfrontmessung sind Beispiele einer objektiven Refraktion.

Unter einer Wellenfrontdarstellung wird eine Parametrisierung einer 2-dimensionalen Wellenfront im 3-dimensionalen Raum verstanden. Darunter fallen insbesondere die folgenden Parametrisierungen:

Polardarstellung (mit den Komponenten Sphäre, Zylinder und Achse), Krümmungsmatrix-Darstellung, Powervektor-Darstellung (mit den Komponenten M, J0 und J45),
Harris-Vektor-Darstellung, Zernike-Polynom Zerlegung (Komponenten sind hier die Zernike-Koeffizienten).

Unter einer objektiven Refraktion wird eine Bestimmung oder die durch die Bestimmung gewonnenen Messwerte der Fehlsichtigkeit eines Auges verstanden, wobei die mit einem während der objektiven Refraktion verwendeten Messgerät vermessene Person die Sehqualität des betrachteten Bildes nicht beurteilen muss. Objektive Refraktionen bzw. objektive Messwerte können z.B. mit Hilfe von Wellenfrontscannern oder Autorefraktometern gemessen werden.

Unter einer subjektiven Refraktion versteht man die Bestimmung oder die Messwerte der Fehlsichtigkeit eines Auges, wobei die refraktionierte Person die Sehqualität des betrachteten Bildes beurteilen muss oder eine Sehaufgabe, z.B. das Erkennen von Optotypen, zu lösen hat und die Lösung mitteilen muss. Subjektive Refraktionen können z.B. von Fachkundigen mit Hilfe von Refraktionsbrillen, in die Refraktionsgläser eingesetzt werden, oder mit Hilfe von Phoroptern erstellt werden. Eine subjektive Refraktion kann auch einen subjektiv bestimmten Nahzusatz, die sogenannte Addition, umfassen.

Der Bezugspunkt ist der Durchblickpunkt eines Brillenglases, in dem die Wirkung des Brillenglases durch die Position und Orientierung des Brillenglases vor dem Auge und durch die Fehlsichtigkeit des Auges, für welches das Brillenglas verwendet werden soll, vorgegeben ist. Dies kann der Fernbezugspunkt, der Prismenbezugspunkt, der Zentrierpunkt, das Zentrierkreuz, der Nachbezugspunkt, etc. sein. Bezüglich der Definition des Bezugspunkts wird auf die Normen DIN EN ISO 21987 (insbesondere Punkt 3.5 bis 3.11) und DIN EN ISO 13666 (insbesondere Punkt 5.12 bis 5.17) verwiesen.

Hinsichtlich der verwendeten Fachterminologie wird insbesondere auf WO 2009/007136 A1, die Publikation von L. Thibos et al., Journal of Vision April 2004, Vol.4, 9. doi:10.1167/4.4.9 und die Publikation Iskander et al., Ophthal. Physiol. Opt. 2007 27:245-255 verwiesen, deren entsprechende Ausführungen insoweit einen integralen Offenbarungsbestandteil der vorliegenden Anmeldung darstellen.

Ein erstes Beispiel betrifft ein Verfahren zum Bestimmen der Fehlsichtigkeit eines Auges eines Brillenträgers, umfassend:

Bereitstellen von Messwerten aus einer ersten und einer zweiten Messung der Fehlsichtigkeit des Auges des Brillenträgers;

Berechnen eines Schätzers bzw. eines Schätzwerts für die Fehlsichtigkeit des Auges des Brillenträgers anhand der Messwerte aus der ersten und der zweiten Messung, wobei bei dem Berechnen des Schätzwerts der Fehlsichtigkeit Messungenauigkeiten bzw. Messabweichungen der ersten Messung und der zweiten Messung der Fehlsichtigkeit berücksichtigt werden.

Sind mehrere Messungen der Fehlsichtigkeit eines Auges bekannt, so können sie gemäß einem Beispiel der Erfindung abhängig von ihren Messungenauigkeiten zur Berechnung eines Schätzers der Fehlsichtigkeit verwendet werden. Vorzugsweise liegt dabei der Schätzer näher bei derjenigen Messung, die die geringere Messungenauigkeit besitzt.

Grundsätzlich können zunächst zwei Arten der Messungenauigkeit unterscheiden werden: Es ist bekannt, dass es systematische Abweichungen gibt, die sich bei Wiederholung einer Messung nicht ändern. Es ist ebenfalls bekannt, dass es sogenannte statistische oder zufällige Abweichungen des Messwerts gibt, die bei Wiederholung einer Messung unterschiedliche Werte annehmen können und sich nicht vorhersagen lassen.

Die Berechnung des Schätzers bzw. des Schätzwerts der Fehlsichtigkeit besteht deshalb darin, die systematischen Abweichungen der Messungen bei der Ermittlung des Schätzers bzw. des Schätzwerts der Fehlsichtigkeit zu berücksichtigen. In diesem Fall kann der mit der systematischen Abweichung behaftete Messwert um diese systematische Abweichung in Richtung der anderen Messung korrigiert werden. Berechnet man danach den Schätzer der Fehlsichtigkeit aus dem korrigierten Messwert der mit systematischen Fehlern behafteten Messung und dem Messwert der nicht durch systematischen Fehlern behafteten Messung, z.B. mit Hilfe eines Mittelwerts, so liegt der Schätzer näher an dem nicht durch systematischen Fehlern behafteten Messwert.

Eine weitere Möglichkeit der Berechnung des Schätzers der Fehlsichtigkeit besteht darin, die statistischen Abweichungen der Messungen bei der Ermittlung des Schätzers der Fehlsichtigkeit zu berücksichtigen. Dies kann vorzugsweise mit Hilfe eines gewichteten Mittelwerts erfolgen. Dabei werden die Gewichte vorzugsweise so gewählt, dass die weniger ungenaue Messung das höhere Gewicht erhält. So können bei normalverteilten Größen die Gewichte proportional zur reziproken Varianz der Messgröße gewählt werden. In Fällen, bei denen keine Normalverteilung vorliegt, kann eine Wahl der Gewichte anhand von Erfahrung notwendig sein. Die weniger genaue Messung kann z.B. Gewichte von 0,3, 0,2, 0,1, 0,05, 0,01 oder weniger bekommen, bis hin zum Gewicht 0. Die genauere Messung kann ein Gewicht von 0,7, 0,8, 0,9, 0,95, 0,99 oder mehr bekommen, bis hin zum Gewicht 1. Sind beide Messungen ähnlich genau, so können sie jeweils ein Gewicht von 0,5 erhalten. Die Gewichte können so gewählt werden, dass ihre Summe 1 beträgt. In diesem Fall ist das Teilen durch die Summe der Gewichte bei der Bildung des gewichteten Mittelwerts nicht mehr notwendig.

Da bei der Messung der Fehlsichtigkeit auch größere statistische Abweichungen auftreten können, z.B. durch Akkommodation, Fluktuationen des Akkommodationszustands, Linsentrübung, Sehschärfe aber auch anderen Größen, kann es vorteilhaft sein, die Gewichte abhängig von der Differenz der Messwerte der Fehlsichtigkeit zu wählen. Zum Beispiel bei Personen, die kaum akkommodieren können und deshalb eine Addition von 1,75 Dpt, 2,0 Dpt, 2,25 Dpt, 2,5 Dpt oder höher verschrieben bekommen haben, sollten sich die subjektiven und objektiven Messwerte des sphärischen Äquivalents kaum unterscheiden. Liegt ein geringer Unterschied vor, so können die sphärischen Äquivalente aus den subjektiven und objektiven Messungen gewichtet addiert werden, wobei mögliche Gewichte des subjektiven und objektiven sphärischen Äquivalents zwischen 0,3 und 0,7 sinnvoll sind. Gibt es aber größere Unterschiede, so ist eher der subjektiven Refraktion zu trauen, da die Person während der subjektiven Refraktion sich bereits ein Bild der Sehqualität durch ein solches Glas machen konnte. In diesen Fall sind für die subjektive Refraktion höhere Gewichte (z.B. 0,8, 0,9, 0,95, 0,99, oder höher, oder sogar 1) zu wählen.

Bei presbyopen Personen, die noch relativ viel akkommodieren können, d.h. Personen, die eine Addition von 1,5 dpt, 1,25 Dpt, 1,0 Dpt, 0,75 Dpt, 0,5 Dpt oder niedriger verschrieben bekommen haben, oder aber Personen, die nicht presbyop sind, d.h. effektiv eine Addition von 0 besitzen, kann z.B. verstärkt Gerätemyopie auftreten. In diesem Fall ist bei einem im Vergleich zum sphärischen Äquivalent der subjektiven Refraktion myoperen sphärischen Äquivalent der objektiven Refraktion letzteres geringer zu gewichten, z.B. mit Gewichten von 0,3, 0,2, 0,1, 0,05, 0,01 oder weniger bis zum Gewicht 0. Sind jedoch die sphärischen Äquivalente der subjektiven Refraktion ähnlich, so bietet es sich an, eine Gewichtung wie bei Presbyopen mit hohen Additionen zu wählen. Ist das subjektive sphärische Äquivalent myoper als das objektive sphärische Äquivalent, z.B. um 0,5 Dpt, könnte die Person bei der subjektiven Refraktion akkommodiert haben. Typischerweise würde hier wieder ein niedrigeres Gewicht für das subjektive sphärische Äquivalent gewählt werden müssen, da aber bei der objektiven Refraktion häufig Gerätemyopie auftreten kann, kann das Gewicht des subjektiven sphärischen Äquivalents auch etwas höher, z.B. zwischen 0,4 und 0,6 gewählt werden.

In der Praxis treten sowohl systematische als auch statistische Abweichungen von Messwerten der Fehlsichtigkeit auf. Vorzugsweise werden in diesem Fall zunächst die systematischen Abweichungen korrigiert, und danach anhand der statistischen Messunsicherheit die korrigierten Messwerte gewichtet kombiniert.

Auch in diesem Fall liegt der so berechnete Schätzer bzw. Schätzwert der Fehlsichtigkeit näher an dem Messwert, dessen Messungenauigkeit geringer ist.

Ein beispielhaftes Verfahren zum Bestimmen einer Fehlsichtigkeit eines Brillenträgers umfasst die folgenden Schritte:

　　1) Angleich der subjektiven und/oder objektiven Refraktionen, um systematische Unterschiede beider Messmethoden zu beseitigen;
　　2) Kombination der so aneinander angeglichenen Refraktionen durch Bildung eines gewichteten Mittelwerts.

**Schritt 1** - **Angleich der subjektiven und objektiven Refraktionen**

Quantifizieren der systematischen Unterschiede zwischen subjektiver und objektiver Refraktion

Um die subjektive und objektive Refraktion aneinander anzugleichen, bzw. die systematischen Unterschiede zwischen subjektiver und objektiver Refraktion zu kompensieren, werden diese zuerst quantifiziert. Dazu muss zunächst ein genügend großer Datensatz vorhanden sein und aufbereitet werden, wie im Folgenden beschrieben ist.

Die systematischen Unterschiede zwischen subjektiver und objektiver Refraktion werden vorzugsweise für den Objektabstand Unendlich quantifiziert, d.h. für die sogenannte Fernverordnung, da diese hinsichtlich des sphärischen Äquivalents wesentlich genauer ist als die Nahrefraktion.

Typischerweise sind ferner die systematischen Unterschiede für verschiedene Gerätemodelle (z.B. Aberrometermodelle) verschiedener Hersteller, unterschiedlich. Es ist deshalb von Vorteil, die Information über das Gerätemodell bereits bei der Datenbeschaffung zu berücksichtigen und die Bestimmung der systematischen Unterschiede für jedes Aberrometermodell separat vorzunehmen.

Um aus der mit einem Gerät (wie z.B. einem Aberrometer, einem Wellenfrontscanner, etc.) gemessenen Wellenfront eine objektive Refraktion zu berechnen, wird vorzugsweise anhand einer sogenannten Metrik die Wellenfront zweiter Ordnung bestimmt. Mögliche Metriken sind z.B. in L. Thibos et al., Journal of Vision April 2004, Vol.4, 9. doi:10.1 167/4.4.9 beschrieben. Es sind jedoch ohne Weiteres auch andere Metriken denkbar und einem Fachmann bekannt.

Die objektiven Refraktionsdaten können z.B. mit Hilfe der *refractive RMS* Metrik von Iskander et al. (Iskander et al., Ophthal. Physiol. Opt. 2007 27:245-255) aus den Wellenfronten bis zur 7. radialen Ordnung berechnet werden, nachdem die Zernike-Wellenfront zentral auf die photopische Pupille skaliert wurde, welche bei der Topographie-Messung der verwendeten Geräte (wie z.B. Aberrometer) gemessenen wurde.

Es ist von Vorteil, den Pupillendurchmesser, bei dem die subjektive Refraktion durchgeführt wurde, zu kennen, wobei dies durch direkte Messung, Schätzung aus anderen Messparametern oder auf Erfahrung gestützte Abschätzung erfolgen kann. Ist der Pupillendurchmesser bekannt, so kann die objektiv gemessene Wellenfront zunächst auf diesen Pupillendurchmesser skaliert werden, und danach der zur Pupille dazugehörige Powervektor berechnet werden. Es ist ebenfalls von Vorteil, bei der Skalierung der Wellenfront die Position der Pupille zu berücksichtigen, falls sich diese bei der Wellenfrontmessung und der subjektiven Refraktion unterscheidet.

Ist der Pupillendurchmesser bei der subjektiven Refraktion unbekannt, so kann er z.B. aus der bei der subjektiven Refraktion auf das Auge fallenden Beleuchtungsstärke und - falls vorhanden - auch anderen Größen wie dem größten (bei schwacher Beleuchtung) und kleinsten (bei starker Beleuchtung) ermittelten Pupillendurchmesser der refraktionierten Person als Schätzwert bestimmt werden.

Die subjektive und objektive Refraktion werden vorzugsweise bei einer für alle Daten identischen Entfernung zum Auge verglichen. Diese Entfernung kann beliebig sein. Als vorteilhaft hat sich jedoch erwiesen, die subjektive Refraktion zunächst auf die Entfernung zum Auge umzurechnen, bei der sich auch die vom Aberrometer, Wellenfrontscanner, etc. gemessenen Wellenfronten befinden. Auf diese Weise erspart man sich die komplexe Propagation der oft Aberrationen höherer Ordnung enthaltenden objektiven Wellenfront. Mögliche sinnvolle Entfernungen zum Auge sind z.B. der Hornhautscheitel oder die Eintrittspupille. Die in den Figuren dargestellten Daten sind für Wellenfronten bzw. Refraktionen am Hornhautscheitel angegeben.

Prinzipiell ist es aber auch möglich, die objektive Refraktion auf eine andere Entfernung vom Auge umzurechnen, wobei auch die Aberration(en) höherer Ordnung enthaltende Wellenfront korrekt propagiert werden muss.

Um möglichst viele Daten mit einem einheitlichen Modell analysieren zu können, können sowohl subjektive als auch objektive Refraktionen der linken Augen vertikal gespiegelt werden. Verwendet man Powervektoren, so muss dafür das Vorzeichen der $J_{45}$-Powervektor-Komponente umgedreht werden. Da die Verteilungen der Aberrationen höherer Ordnung bei linken und rechten Augen spiegelsymmetrisch sind, können auf diese Weise die Refraktionen der rechten und der gespiegelten linken Augen zusammen analysiert werden.

Es kann aber auch von Vorteil sein, diese Spiegelung nicht vorzunehmen, z.B. wenn das Aberrometer eine gespiegelte Wellenfront nicht korrekt messen würde. In diesem Fall sind die Korrekturen für linke und rechte Augen separat auszuwerten und vorzunehmen.

Es ist ebenfalls von Vorteil, zum Quantifizieren der systematischen Unterschiede zwischen subjektiver und objektiver Refraktion nur den Teil der Datensätze auszuwerten, der keine oder nur wenige Artefakte besitzt. Mögliche Artefakte sind z.B. Gerätemyopie oder altersbedingte Augenkrankheiten. So kann z.B. nur der Teil der Daten verwendet werden, bei dem sich die verordnete Addition vom reziproken Objektabstand bei der Nahrefraktion nur wenig unterscheidet, d.h. der Betrag der Differenz aus Addition und reziprokem Objektabstand (bei positiver Vorzeichenkonvention) darf nicht größer als ein vorbestimmter Schwellenwert sein. Der Schwellenwert kann z.B. 0 Dpt, 0,25 Dpt oder 0,5 Dpt sein. Dies verkleinert die Anzahl und das Ausmaß der Gerätemyopie im Datensatz.

Eine andere Einschränkung ist die auf eine hohe Sehschärfe, was Artefakte durch Refraktion von amblyopen Personen oder andere Anomalien des zentralen Sehens vermeidet. So kann nur der Teil der Daten verwendet werden, bei dem die Sehstärke höher als ein vorbestimmter Schwellenwert ist. Mögliche Grenzen sind hier z.B. 1,25 oder 1,6 oder mehr bei einer dezimalen Sehschärfe monokular. Vorzugsweise wird diese Bedingung bei beiden Augen erfüllt.

Es ist ebenfalls vorteilhaft, nur Daten von Refraktionierenden zu verwenden, die bei der subjektiven Refraktion Refraktionsgläser mit genügend hoher Auflösung verwenden (z.B. 0,25 Dpt oder vorzugsweise 0,125 Dpt bei sphärischen Refraktionsgläsern, und 0,5 Dpt oder noch besser 0,25 Dpt bei Zylindergläsern). Dies kann aus der Verteilung der Glasbestellungen des jeweiligen Refraktionierenden ermittelt werden.

Zum Anpassen der Daten wird vorzugsweise zunächst ein Modell der subjektiven Refraktion aufgestellt, mit dem aus der gemessenen objektiven Refraktion und ggf. anderen Messgrößen eine vorhergesagte Refraktion berechnet werden kann, und mit welchem Abweichungen der tatsächlich gemessenen subjektiven Refraktion zur vorhergesagten objektiven Refraktion statistisch quantifiziert werden können. Dieses Modell wird in einem nachfolgenden Schritt an die Daten angepasst.

Es ist von Vorteil, die Anpassung des Modells an die subjektive Refraktion im Powervektorraum vorzunehmen (siehe L. Thibos et al.: Power Vectors: An Application of Fourier Analysis to the Description and Statistical Analysis of Refractive Error, Optometry and Vision Science 74, 6, 367-375), und dazu die gemessenen und sich systematisch unterscheidenden subjektiven und objektiven Refraktionen als Powervektoren der subjektiven Refraktion $\tilde{P}_{sub} = (\tilde{M}_{sub},$
$\widetilde{J0}_{sub}, \widetilde{J45}_{sub})$ und der objektiven Refraktion $\tilde{P}_{obj} = (\tilde{M}_{obj}, \widetilde{J0}_{obj}, \widetilde{J45}_{obj})$ anzugeben. Die Tilde in der Notation bezieht sich dabei auf die nicht korrigierten (rohen) Daten im Datensatz.

Im Rahmen des Modells wird die tatsächlich gemessene subjektive Refraktion anhand der vorhergesagten Refraktion, $P_{pred} = (M_{pred}, J0_{pred}, J45_{pred})$, und den Zufallsvariablen $\varepsilon_M$, $\varepsilon_{J0}$ und $\varepsilon_{J45}$ beschrieben, wobei letztere sowohl die Messungenauigkeit des Geräts als auch die der refraktionierenden Person modellieren:

$$\tilde{M}_{sub} = M_{pred} + \varepsilon_M$$
$$\widetilde{J0}_{sub} = J0_{pred} + \varepsilon_{J0}$$
$$\widetilde{J45}_{sub} = J45_{pred} + \varepsilon_{J45}.$$

$$(1)$$

Als Kurzschreibweise für dieses Gleichungssystem wird im Folgenden

$$\tilde{P}_{sub} = P_{pred}[\tilde{P}_{obj}, ...] + \varepsilon$$

$$(1a)$$

verwendet, wobei $\varepsilon$ der Powervektor $(\varepsilon_M, \varepsilon_{J0}, \varepsilon_{J45})$ ist. Gleichung 1a gilt für jede Messung des Datensatzes, sodass für die i-te Messung

$$\tilde{P}^i_{sub} = P_{pred}[\tilde{P}^i_{obj}, ...] + \varepsilon^i$$

$$(1b)$$

geschrieben werden kann.

Der Powervektor der vorhergesagten Refraktion $P_{pred}$ hängt von der objektiven Refraktion ab. Gegebenenfalls kann er auch von zusätzlichen Größen wie z.B. von der subjektiven Refraktion abhängen, oder z.B. von Pupillendurchmessern, oder anderen Messgrößen, die während einer Refraktion oder einer objektiven Messung (z.B. einer Aberrometer-Messung) anfallen.

Ein Kriterium, anhand dessen das Modell an die Daten angepasst werden kann, ist das Maximieren der Wahrscheinlichkeits(dichte) der Zufallsgrößen $\varepsilon_M$, $\varepsilon_{J0}$ und $\varepsilon_{J45}$ im Parameterraum des Modells bei eingesetztem Datensatz, was im Folgenden auch "Fit" genannt wird. Geeignete Methoden sind z.B. "maximum Likelihood" Methoden, welche die Wahrscheinlichkeit den anzupassenden Datensatz zu generieren, die sog. Likelihood, maximiert. In den hier offenbarten Modellen ist die Likelihood durch die folgende Gleichung gegeben

$$prob(\{\varepsilon_X\}; Parameter) = \prod_i prob(\varepsilon_X^i; Parameter),$$

$$(2)$$

wobei *prob({ε_X}; Parameter)* die Wahrscheinlichkeitsdichte des gesamten Datensatzes bei vorliegenden Parametern des Modells ist, und $prob(\varepsilon_X^i; Parameter)$ die Wahrscheinlichkeitsdichte einer einzelnen Messung *i* aus dem Datensatz ist.

Als eine mögliche Alternative zur Maximum Likelihood Methode kann die Methode der kleinsten Quadrate verwendet werden, welche auch als äquivalent zur "maximum likelihood" Methode mit einer normalverteilten Likelihood angesehen werden kann. In den Modellen kann ferner auch Vorwissen über die verwendeten Parameter einfließen, was im Sinne der Bayes'schen Datenanalyse möglich ist.

Die Zufallsvariable $\varepsilon_X$ der Messungenauigkeit der Powervektor-Komponente *X* (wobei X für M, $J_0$ oder $J_{45}$ steht) kann z.B. durch eine Überlagerung einer Gleichverteilung, genannt auch uniforme Verteilung, (z.B. im Bereich -20 bis +20 Dpt oder in einem anderen geeigneten Wirkungsbereich) und einer Voigt-Verteilung mit der Gauss-Breite $\sigma^X$ (als Standardabweichung) und der Lorentz-Breite $\gamma^X$ (als Halbwertsbreite) beschrieben werden. Die uniforme Verteilung kann große "Ausreißer" in den Daten beschreiben, welche mit der Wahrscheinlichkeit $p_0^X$ auftreten. Die Voigt-Verteilung, die mit der Wahrscheinlichkeit $1 - p_0^X$ auftritt, beschreibt eine erfolgreiche Messung, welche jedoch auch moderate Ausreißer erzeugen kann. Insgesamt können die Zufallsvariable $\varepsilon_X$ also folgendermaßen verteilt sein:

$$prob(\varepsilon_X^i; Parameter) = prob(\varepsilon_X^i; p_0^X, \sigma^X, \gamma^X) = p_0^X unif(\varepsilon_X^i; min = -20, max = 20) +$$
$$(1 - p_0^X)Voigt(\varepsilon_X^i; \sigma^X, \gamma^X).$$

$$(3)$$

Alternativ zur Voigt-Verteilung kann auch die Normalverteilung gewählt werden, mit der jedoch schlechtere Ergebnisse erzielt werden. Wichtig ist vor allem der Term mit der uniformen Verteilung, da er in der Lage ist, große Ausreißer abzufangen.

Zur Berechnung der vorhergesagten Refraktion können beispielsweise als Eingabegrößen nur die Powervektor-Komponenten der objektiven Refraktion verwendet werden. Die Berechnung kann dabei anhand einer beliebigen parametrisierbaren Funktion, wie zum Beispiel mit Hilfe von Polynomen, stattfinden. Ein beispielhaftes Modell ist das durch das Gleichungssystem (2) beschriebene Modell **(Modell 1)**:

$$M_{pred}(\widetilde{M}_{obj}, \widetilde{J0}_{obj}, \widetilde{J45}_{obj}) = \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj}$$

$$J0_{pred}(\widetilde{M}_{obj}, \widetilde{J0}_{obj}, \widetilde{J45}_{obj}) = a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj}$$

$$J45_{pred}(\widetilde{M}_{obj}, \widetilde{J0}_{obj}, \widetilde{J45}_{obj}) = a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i}$$

$$(4)$$

Dabei sind die $a_{X,i}^{Y}$ die Modellparameter für den Fit der Powervektor-Komponente *Y* der subjektiven Refraktion, die mit den objektiven Powervektor-Komponenten *X* der objektiven Refraktion interagiert. Hier steht *Y* für $M_{pred}$, $J0_{pred}$ oder $J45_{pred}$ und *X* für $\widetilde{M}_{obj}, \widetilde{J0}_{obj}, \widetilde{J45}_{obj}$.

Die einzelnen Powervektor-Komponente $M_{pred}$, $J0_{pred}$ oder $J45_{pred}$ der vorhergesagten Refraktion sind vorzugsweise Funktionen aller drei Komponenten des Powervektors der gemessenen (rohen) objektiven Refraktion.

In einem alternativen Modell wird zusätzlich noch die Information aus den nicht gefitteten Komponenten des subjektiven Powervektors benutzt, um die vorhergesagte Refraktion zu berechnen. Die Berechnung kann dabei anhand einer beliebigen parametrisierbaren Funktion, wie zum Beispiel mit Hilfe von Polynomen, stattfinden. Ein beispielhaftes Modell ist das durch das Gleichungssystem (3) beschriebene Modell **(Modell 2):**

$$M_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj} + b_{J0,1}^{M} \widetilde{J0}_{sub} + b_{J45,1}^{M} \widetilde{J45}_{sub}$$

$$J0_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj} + b_{M,1}^{J0} \widetilde{M}_{sub} + b_{J45,1}^{J0} \widetilde{J45}_{sub}$$

$$J45_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i} + b_{M,1}^{J45} \widetilde{M}_{sub} + b_{J0,1}^{J45} \widetilde{J0}_{sub}$$

$$(5)$$

Durch die mögliche Interaktion von subjektiven und objektiven Powervektor-Komponenten können auch Fehler bei der subjektiven Refraktionsbestimmung modelliert bzw. berücksichtigt werden. So können Fehler die auf Refraktionspraktiken, wie z.B. das Verändern des Zylinders bei gleichbleibender Sphäre, modelliert werden, die aus Mangel an Auflösung bei den Refraktionsgläsern oder aus Unwissen des Augenoptikers entstehen können.

Alternativ zu den oben beschriebenen "maximum Likelihood" Modell können auch z.B. Schätzer der subjektiven Refraktion wie der laufende Median des Powervektors der subjektiven Refraktion berechnet werden. Eine parametrisierbare Beschreibung des vorhergesagten Powervektors, wie z.B. mittels Gleichungssystem 2 oder 3, kann dann mittels der Methode der kleinsten Quadrate an den berechneten Schätzer der subjektiven Refraktion angepasst werden. Natürlich können auch andere Schätzer als der Mittelwert oder Median verwendet werden, sofern deren Fehler näherungsweise normalverteilt sind, bevor die Methode der kleinsten Quadrate verwendet wird. Dagegen ist die Verwendung des reinen Mittelwertes oder der direkten Anpassung der Daten mit der Methode der kleinsten Quadrate aufgrund möglicher Ausreißer in den Daten nicht vorteilhaft.

Beispiele der zu den Modellen 1 und 2 gehörenden Parametersätze sind in Tabelle 1 und 2 dargestellt. Die Tabellen 1 und 2 zeigen die Fit-Ergebnisse für zwei unterschiedliche Aberrometer (Aberrometer 1 und Aberrometer 2).

Die Parameter $a_{Y,i}^{X}$ quantifizieren die systematischen Abweichungen, die anderen Parameter quantifizieren die Messunsicherheiten der Aberrometer. Das Symbol "*" in den Tabellen 1 und 2 bedeutet, dass die entsprechende Variable die abhängige Variable ist, die vorhergesagt werden sollte, es gibt also keinen entsprechenden Parameter. Insbesondere beziehen sich die Sternchen darauf, dass es keinen entsprechenden Parameter gibt, ansonsten wäre die Lösung von Gleichung 1a trivial erfüllt mit dem fehlenden Parameter = 1 und allen anderen =0).

Die Tabelle 1 enthält die Fit-Ergebnisse des Modells ohne zusätzlichen Einfluss von subjektiven Powervektor-Komponenten (Modell 1). Die Tabelle 2 enthält die Fit-Ergebnisse des Modells mit zusätzlichem Einfluss von subjektiven Powervektor-Komponenten (Modell 2).

Tabelle 1

| | Aberrometer 1 | | | Aberrometer 2 | | |
|---|---|---|---|---|---|---|
| X | M | J0 | J45 | M | j0 | J45 |
| $ln(p_0^X)$ | -5,988559e+00 | -14,683359254 | -1,030284e+01 | -8,260018e+00 | -9,102539677 | -1,849467e+01 |
| $ln(\sigma^X)$ | -1,473851e+00 | -2,260253864 | -2,399429e+00 | -1,484472e+00 | -2,257872473 | -2,412253e+00 |

(fortgesetzt)

| | Aberrometer 1 | | | Aberrometer 2 | | |
|---|---|---|---|---|---|---|
| X | M | J0 | J45 | M | j0 | J45 |
| $ln(\gamma^X)$ | -3,708547e+00 | -4,014985072 | -4,173024e+00 | -3,429828e+00 | -3,995043504 | -4,137689e+00 |
| $a_{X,0}^X$ | 2,597766e-02 | -0,005823866 | 1,167734e-03 | -8,455234e-02 | -0,002214542 | -1,396330e-03 |
| $a_{M,1}^X$ | 9,553421e-0 1 | 0,003854467 | 3,013793e-04 | 9,448156e-01 | 0,003801166 | 8,891828e-04 |
| $a_{J0,1}^X$ | -1,089862e-02 | 0,884505067 | -2,097478e-02 | -2,125544e-02 | 0,880071958 | -1,653286e-02 |
| $a_{J45,1}^X$ | -2,055721e-02 | 0,018582216 | 8,162184e-01 | -1,948651e-02 | 0,012354087 | 8,022532e-0 1 |
| $a_{X,2}^X$ | -7,955925e-03 | 0,033536394 | 8,674891e-03 | -9,785492e-03 | 0,029741058 | 4,053460e-02 |
| $a_{X,3}^X$ | -7,773469e-05 | 0,010410193 | 4,275422e-02 | 4,552031e-05 | 0,016098649 | 3,814216e-02 |
| $a_{X,4}^X$ | 7,617699e-05 | -0,006240530 | -5,976109e-03 | 1,110717e-04 | -0,00325121 1 | -1,156952e-02 |

Tabelle 2

| | Aberrometer 1 | | | Aberrometer 2 | | |
|---|---|---|---|---|---|---|
| X | M | J0 | J45 | M | J0 | J45 |
| $ln(p_0^X)$ | -1,158685e+01 | -23,634670622 | -1,801281e+01 | -9,917428e+00 | -13,824630577 | -14,15287304 |
| $ln(\sigma^X)$ | -1,478691e+00 | -2,255383300 | -2,397918e+00 | -1,490140e+00 | -2,257999967 | -2,41899596 |
| $ln(\gamma^X)$ | -3,645794e+00 | -4,058939935 | -4,179863e+00 | -3,451811e+00 | -4,0312991 12 | -4,12621854 |
| $a_{X,0}^X$ | 3,849776e-02 | -0,005832360 | 1,195480e-03 | -9,787282e-02 | -0,010296598 | 0,00105432 |
| $a_{M,1}^X$ | 9,530755e-01 | 0,049540280 | 7,342407e-04 | 9,492652e-01 | 0,057330004 | -0,01627960 |
| $a_{J0,1}^X$ | 2,150168e-01 | 0,884831027 | -3,017279e-02 | 2,295930e-01 | 0,878258986 | -0,03860188 |
| $a_{J45,1}^X$ | -3,884270e-03 | -0,004837432 | 8,160447e-01 | -1,549439e-01 | 0,008366583 | 0,80318242 |
| $b_{M,1}^X$ | * | -0,047771141 | -4,900606e-04 | * | -0,056451915 | 0,01814338 |
| $b_{J0,1}^X$ | -2,406537e-01 | * | 1,039228e-02 | -2,730474e-01 | * | 0,02477013 |
| $b_{J45,1}^X$ | -8,299017e-03 | 0,027505589 | * | 1,467214e-01 | 0,003420205 | * |
| $a_{X,2}^X$ | -8,175954e-03 | 0,031644005 | 8,539891e-03 | -9,243794e-03 | 0,032050577 | 0,03769131 |
| $a_{X,3}^X$ | 3,245316e-05 | 0,009592653 | 4,276131e-02 | -2,466715e-05 | 0,016408827 | 0,03787041 |
| $a_{X,4}^X$ | 7,550834e-05 | -0,005829236 | -5,949418e-03 | 1,072269e-04 | -0,003381591 | -0,01119501 |

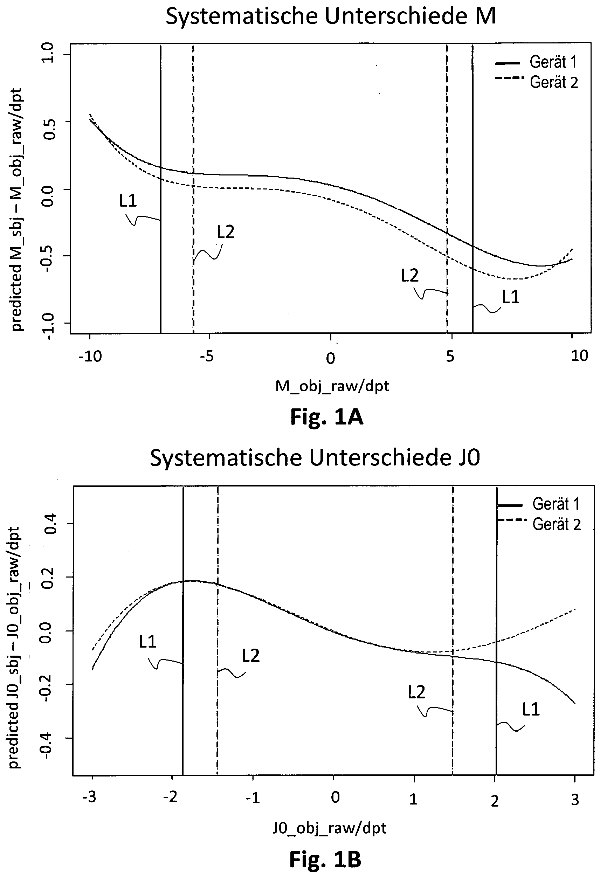

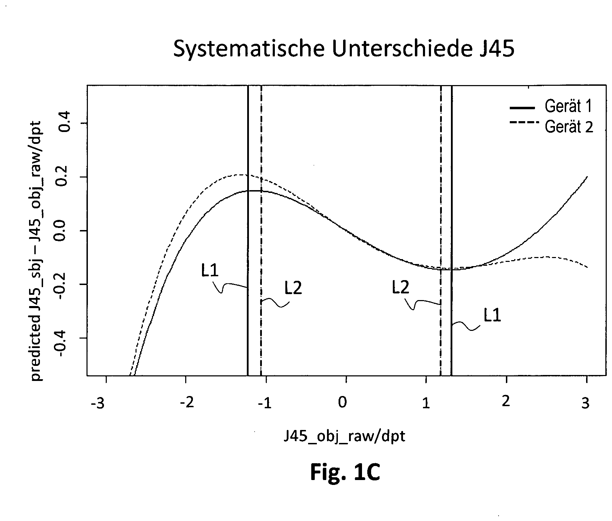

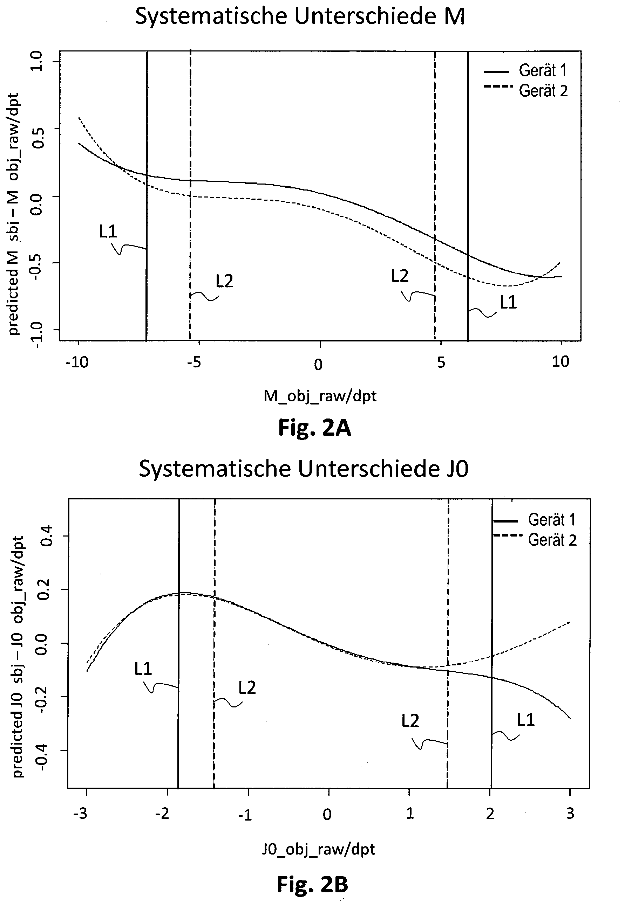

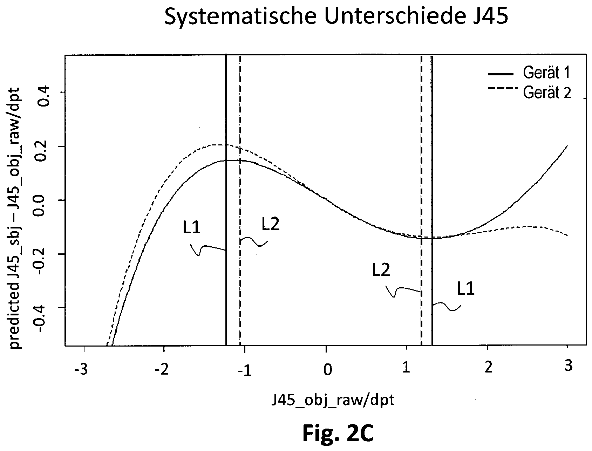

Mit den so parametrisierten Modellen können die systematischen Abweichungen von subjektiver und objektiver Refraktion bei verschiedenen Gerätemodellen (wie z.B. zwei verschiedenen Aberrometermodellen) korrigiert werden. Der Verlauf der jeweiligen Powervektor-Komponenten *X* bei auf 0 gesetzten objektiven Powervektor-Komponenten Y≠X ist in **Figuren 1** und **2** dargestellt.

**Figuren 1** und **2** zeigen die systematischen Abweichungen objektiver und subjektiver Wellenfronten bei zwei verschiedenen Aberrometern (Aberromter 1: durchgezogene Linie, Aberrometer 2: gestrichelte Linie). Jede Powervektor-Komponente wurde mit zwei unterschiedlichen Modellen quantifiziert. Modell 1 (Figuren 1A bis 1C) enthält keinen Einfluss der subjektiven Refraktion, bei Modell 2 (Figuren 2A bis 2B) ist der Einfluss der subjektiven Refraktion enthalten.

**Fig. 1A** und **2A** zeigen die Differenz zwischen dem vorhergesagten Wert (predicted M_sbj) des mittels subjektiver Refraktion ermittelten sphärischen Äquivalents M und dem mittels objektiver Refraktion gemessenen Wert (M_obj_raw) des sphärischen Äquivalents als Funktion des mittels objektiver Refraktion gemessenen Messwerts (M_obj_raw) des sphärischen Äquivalents M.

**Fig. 1B** und **2B** zeigen die Differenz zwischen dem vorhergesagten Wert (predicted J0_sbj) der Komponente J0 des Powervektors der subjektiven Refraktion und dem mittels objektiver Refraktion ermittelten, gemessenen Wert (J0_obj_raw) der Komponente J0 als Funktion des mittels objektiver Refraktion ermittelten gemessenen Werts (J0_obj_raw) der Komponente J0.

**Fig. 1C** und **2C** zeigen die Differenz zwischen dem vorhergesagten Wert (predicted J0_sbj) der Komponente J45 des Powervektors der subjektiven Refraktion und dem mittels objektiver Refraktion ermittelten, gemessenen Wert (J45_obj_raw) der Komponente J0 als Funktion des mittels objektiver Refraktion ermittelten, gemessenen Werts (J45_obj_raw) der Komponente J0.

Mit den vertikalen Linien L1 (Modell 1) und L2 (Modell 2) sind die Bereiche gekennzeichnet, an denen sich im Datensatz eine genügend hohe Dichte an Daten, hier etwa von 50 Augen (links + rechts) pro Dioptrie der jeweiligen Powervektor-Komponente befinden.

Wie aus den Figuren 1 und 2 ersichtlich, ist bei beiden Aberrometermodellen sowohl bei dem ersten als auch bei dem zweiten Modell das sphärische Äquivalent bei Hyperopen subjektiv niedriger als objektiv. Bei Myopen ist das umgekehrt, und nicht so stark ausgeprägt. Insgesamt ist also bei der subjektiven Refraktion eine vom Betrag her schwächere Korrektion zu sehen. Dies ist auch bei den astigmatischen Powervektor-Komponenten der Fall.

Korrektur der systematischen Abweichungen der objektiven Refraktion

Geht man davon aus, dass die objektive Refraktion systematisch falsch ist, kann z.B. anhand des Modells 1 oder des Modells 2, die objektive Refraktion anhand des Modells im Mittel an die subjektive Refraktion angeglichen werden. Dies geschieht indem der beim Anpassen des Modells an einer Vielzahl von Daten bestimmte Powervektor-Unterschied zwischen subjektiver und objektiver Refraktion, Δ*P*, zum Powervektor der objektiven Refraktion $\tilde{P}_{obj}$ hinzuaddiert wird. Wurden die Daten einem in Gleichungssystem 1 beschriebenen Modell angepasst, so ist

$$\Delta P\big[\tilde{P}_{obj}\big] = P_{pred}\big[\tilde{P}_{obj}\big] - \tilde{P}_{obj}.$$

$$(6)$$

Der Unterschied zwischen subjektiver und objektiver Refraktion hängt bei der Verwendung von Modell 1 allein von der objektiven Refraktion ab:

$$P_{obj} = \tilde{P}_{obj} + \Delta P\big[\tilde{P}_{obj}\big]$$

$$(7)$$

Um Überschwinger des Modells und damit eine Fehlkorrektur zu vermeiden, ist es bevorzugt, die Korrekturen Δ*P* auf einen Bereich zu begrenzen, in dem genügend Daten vorliegen:

$$P_{obj} = \tilde{P}_{obj} + \Delta P\big[B\big(\tilde{P}_{obj}\big)\big]$$

$$(8)$$

Dabei begrenzt die Funktion *B*(.) den Powervektor $\tilde{P}_{obj}$ auf den Bereich, in dem genügend Daten vorhanden sind. *B*(.) kann z.B. als eine senkrechte Projektion auf die Seitenflächen eines einfachen Kastens implementiert werden. Außerhalb des Bereichs B kann die Änderung ΔP auf einen konstanten Wert gesetzt werden.

**Tabelle 3** zeigt mögliche Begrenzungen des Gültigkeitsbereichs des Modells. Die Powervektor-Komponente X wird durch die Begrenzungsfunktion B(.) auf max(min(X, Max_X), Min_X) abgebildet. Die Begrenzungen richten sich nach einer Daten-Dichte von 50 Messungen pro Dioptrie der jeweiligen Powervektor-Komponente. Innerhalb des in Tabelle 3 dargestellten Bereichs behebt Schritt 1 die systematischen Unterschiede relativ gut. Außerhalb des Bereichs wird die Änderung ΔP konstant gehalten.

Alternativ können andere Kriterien verwendet werden wie z.B., bezogen auf die Daten-Dichte geteilt durch die Determinante der Jacobi-Matrix der Korrektur $\Delta P[\tilde{P}_{obj}]$ relativ zu dem objektiven Powervektor, d.h. Daten-Dichte / $\det[\partial \Delta P[\tilde{P}_{obj}]/\partial \tilde{P}_{obj}]$.

**Tabelle 3**

|  | Beispiel 1 | | | Beispiel 2 | | |
|---|---|---|---|---|---|---|
| Powervektor-Komponente X | M / Dpt | J0 / Dpt | J45 / Dpt | M / Dpt | J0 / Dpt | J45 / Dpt |
| Min_X | -7,04 | -1,87 | -1,23 | -5,72 | -1,44 | -1,06 |
| Max_X | 5,89 | 2,02 | 1,31 | 4,76 | 1,47 | 1,19 |

Es sind auch weitere Begrenzungsfunktionen denkbar, wie z.B. die Projektion eines beliebigen Punktes im Bereich ohne bzw. mit nur einer geringen Anzahl von Daten auf den Rand einer Iso-Wahrscheinlichkeitsdichte-Fläche der Daten im Raum des nicht korrigierten, objektiven Powervektors. Es ist von Vorteil, wenn die Projektion entlang des Gradienten der Wahrscheinlichkeitsdichte der Daten durchgeführt wird. Die Projektionslinien ergeben sich in diesem Fall durch die Lösung einer linearen Differenzialgleichung, und verlaufen durch den zu projizierenden Powervektor, was ein klassisches Anfangswerteproblem darstellt. Sollte die Dichte der Daten durch eine multidimensionale (z.B. 3-dimensionale) Verteilung beschrieben werden, so kann die Lösung der Differenzialgleichung sogar analytisch erfolgen. Bei anderen Wahrscheinlichkeitsdichtefunktionen kann eine numerische Lösung notwendig sein. Ist erst eine Projektionslinie gefunden, so lässt sich ihr Schnittpunkt mit der zur gewünschten Daten-Dichte gehörenden Iso-Wahrscheinlichkeitsfläche mit Hilfe einer 1-dimensionalen Suche entlang der Projektionslinie numerisch durchführen.

Es sind auch andere Arten von Begrenzungen der Korrektur möglich. Zum Beispiel wäre es denkbar, die Änderung ΔP außerhalb des begrenzten Bereichs nicht konstant zu halten, sondern sich abhängig von der Entfernung des nicht korrigierten, objektiven Powervektors $\tilde{P}_{obj}$ zum Rand der Begrenzung linear ändern zu lassen. Dies entspricht effektiv einem stückweise definierten Modell, das innerhalb der Begrenzung ein Polynom höherer Ordnung ist, und außerhalb der Begrenzung linear verläuft. Dabei ist der Übergang an der Begrenzung so zu wählen, dass das Modell in Bezug auf $\tilde{P}_{obj}$ stetig ableitbar ist.

Im einfachsten Fall wird die subjektive Refraktion nicht korrigiert, und - wie oben dargestellt - alleine die objektive Refraktion an die subjektive Refraktion angeglichen, um die quantifizierten systematischen Unterschiede zwischen beiden Refraktionsmethoden zu kompensieren.

Korrektur der systematischen Abweichungen der subjektiven Refraktion

Es ist aber auch möglich, die subjektive Refraktion an die objektive Refraktion anzupassen. In diesem Fall wird zur Berechnung des Powervektors einer korrigierten subjektiven Refraktion, $P_{sub}$, der mit Hilfe des Modells ermittelte systematische Unterschied ΔP vom Powervektor der ursprünglichen subjektiven Refraktion $\tilde{P}_{sub}$ subtrahiert:

$$P_{sub} = \tilde{P}_{sub} - \Delta P[\tilde{P}_{obj}].$$

$$(9)$$

Dies kann z.B. notwendig sein, wenn die systematischen Unterschiede durch fragwürdige Refraktionstechniken entstehen, z.B. durch Weglassen oder Verändern der Stärke des Zylinder-Refraktionsglases, ohne dass die Sphäre entsprechend um die Hälfte der Änderung der Stärke des Zylinder -Refraktionsglases angepasst wird, um das sphärische Äquivalent konstant zu halten.

Korrektur der systematischen Abweichungen der objektiven und der subjektiven Refraktion

Im Allgemeinen kann es auch von Vorteil sein, den Powervektor der systematischen Unterschiede zwischen subjektiven und objektiven Powervektoren, $\Delta P$, in zwei Teile aufzuspalten, von denen einer, $\Delta P_{obj}$, zur Korrektur des Powervektors der objektiven Refraktion verwendet wird, und der andere, $\Delta P_{sub}$, zur Korrektur der subjektiven Refraktion verwendet wird:

$$\Delta P = \Delta P_{sub}\left[\tilde{P}_{sub}, \tilde{P}_{obj}\right] + \Delta P_{obj}\left[\tilde{P}_{sub}, \tilde{P}_{obj}\right]$$

$$P_{obj} = \tilde{P}_{obj} + \Delta P_{obj}\left[\tilde{P}_{sub}, \tilde{P}_{obj}\right]$$

$$P_{sub} = \tilde{P}_{sub} - \Delta P_{sub}\left[\tilde{P}_{sub}, \tilde{P}_{obj}\right].$$

$$(10)$$

Die Differenzen $\Delta P_{obj}$ und $\Delta P_{sub}$ können dabei sowohl von der nicht korrigierten, objektiven Refraktion, $\tilde{P}_{obj}$, als auch von der nicht korrigierten, subjektiven Refraktion, $\tilde{P}_{sub}$, abhängen. Dabei ist $P_{obj}$ die korrigierte objektive Refraktion, und $P_{sub}$ die korrigierte subjektive Refraktion.

Die Teile der systematischen Unterschiede können vorteilhafter Weise so aufgeteilt werden, dass Terme des Modells, die Differenzen von nicht korrigierten subjektiven und objektiven Powervektor-Komponenten enthalten (z.B. Terme proportional zu $\tilde{J0}_{sub} - \tilde{J0}_{obj}$ oder einer Potenz davon, welche im Modell des sphärischen Äquivalents vorkommen), zur Korrektur der subjektiven Refraktion benutzt werden, da es sich mit hoher Wahrscheinlichkeit um den Effekt einer wie z.B. der oben erwähnten fragwürdigen Refraktionsmethode handelt. Die verbleibenden Terme (z.B. solche, die rein von den Komponenten des Powervektors der nicht korrigierten objektiven Refraktion abhängen) können zur Korrektur der objektiven Refraktion benutzt werden.

Es ist auch möglich, die Korrekturen mit einem für alle Powervektor-Komponenten gemeinsamen reellen Faktor $\alpha$, oder aber mit für jede Powervektor-Komponente unterschiedlichen reellen Faktoren $\alpha, \beta, \gamma$, auf die objektiven und subjektiven Differenzen $\Delta P_{obj}$ und $\Delta P_{sub}$ aufzuteilen:

$$P_{obj} = \tilde{P}_{obj} + \alpha \Delta P_{obj}\left[\tilde{P}_{sub}, \tilde{P}_{obj}\right]$$

$$P_{sub} = \tilde{P}_{sub} - (1 - \alpha)\Delta P_{sub}\left[\tilde{P}_{sub}, \tilde{P}_{obj}\right],$$

bzw.

$$P_{obj} = \tilde{P}_{obj} + \begin{bmatrix} \alpha & 0 & 0 \\ 0 & \beta & 0 \\ 0 & 0 & \gamma \end{bmatrix} \Delta P_{obj}\left[\tilde{P}_{sub}, \tilde{P}_{obj}\right]$$

$$P_{sub} = \tilde{P}_{sub} - \left(1 - \begin{bmatrix} \alpha & 0 & 0 \\ 0 & \beta & 0 \\ 0 & 0 & \gamma \end{bmatrix}\right) \Delta P_{sub}\left[\tilde{P}_{sub}, \tilde{P}_{obj}\right]$$

$$(11)$$

Dies wäre jedoch erst dann sinnvoll, wenn bekannt wäre, dass eine dritte Refraktionsmethode keine oder einfach nur geringere systematische Fehler aufweist als die subjektive Refraktion und die objektive Refraktion, deren systematische Abweichung voneinander bereits quantifiziert wurde. Zudem müsste die systematische Abweichung der subjektiven Refraktion und der objektiven Refraktion von der dritten Refraktionsmethode proportional zu den systematischen Abweichungen zwischen der subjektiven und objektiven Refraktion sein.

Die Berechnung der korrigierten objektiven und subjektiven Refraktionen kann natürlich auch mit einer Begrenzung der Korrektur kombiniert werden.

Werden die korrigierten objektiven Werte der refraktionierenden Person angezeigt, z.B. am Aberrometer oder Autorefraktometer, so ist es möglich, dass die subjektive Refraktion durch das objektive Messergebnis beeinflusst wird. Es kann deshalb auch von Vorteil sein, das oben beschriebene Verfahren zur Bestimmung der Korrektur der systematischen Unterschiede zwischen subjektiver und objektiver Refraktion mehrmals durchzuführen, z.B. mit Datensätzen aus jeweils einem halben Jahr Bestellungen. Auf diese Weise wird die Beeinflussung der subjektiven Refraktion durch die Darstellung der objektiven Refraktion schrittweise vermindert.

Alternativ dazu kann auch ein Modell zur Bestimmung der systematischen Unterschiede gewählt werden, das den Anteil und das Ausmaß der Beeinflussung quantifiziert. Solche Modelle können z.B. anhand von Schätzwerten aufgestellt werden, oder aber durch Auswertung von Studien mit einer relativ geringen Anzahl von refraktionierten und refraktionierenden Personen, von denen ein Teil vor der subjektiven Refraktion eine objektive Refraktion erstellen muss, und der andere Teil keine objektive Refraktion durchführen darf. Es ist auch möglich, dass ein und dieselbe refraktionierende Person subjektive Refraktionen verschiedener Personen sowohl mit als auch ohne eine vorhergehende objektive Refraktion durchführt. Möglich und ggf. ergänzend ist in diesem Fall aber auch der Vergleich zweier Verteilungen einer größeren Menge an Refraktionsdaten (z.B. als Powervektoren), die von Refraktionierenden erstellt wurden, die keine Möglichkeit hatten, eine objektive Refraktion zu messen (erste Verteilung), bzw. von solchen, die zwingend eine objektive Refraktion erstellt haben (zweite Verteilung). Solche Datensätze fallen in großer Menge beim Bestellprozess von Brillengläsern an und können relativ einfach gewonnen und untersucht werden, ohne dass spezielle Studien notwendig sind. Wird ein solches Modell verwendet, so ist eine Wiederholung des Verfahrens zur Bestimmung der Korrektur nicht zwingend notwendig.

Anstatt die systematischen Fehler der subjektiven und objektiven Refraktionen als Powervektoren aneinander anzugleichen, können selbstverständlich auch andere Darstellungen von Refraktionsfehlern verwendet werden, wie zum Beispiel Sphäre, Zylinder, Achse oder die Zernike-Zerlegung von Wellenfronten.

Bei einer Darstellung als Wellenfront wird die subjektive Refraktion, vorzugsweise bei dem während der Refraktion vorliegenden Pupillendurchmesser, in eine Wellenfront umgewandelt (subjektive Wellenfront). Verfahren zur Umwandlung in eine Wellenfront sind aus dem Stand der Technik bekannt. Dabei könnten auch die durch die Refraktionsgläser erzeugten Aberrationen höherer Ordnung berücksichtigt werden, selbst wenn diese im Allgemeinen niedrig sind. Danach kann ein Modell zur Vorhersage der subjektiven Wellenfront aus der objektiven Wellenfront an die vorliegenden Daten, d.h. an die subjektiven und objektiven Wellenfronten, angepasst werden. Dabei kann es vorteilhaft sein, die Darstellungen wie z.B. Zernike-Koeffizienten, vor der Analyse geeignet zu normieren. Die zum Angleich verwendete Korrektur ergibt sich analog zum oben beschriebenen Verfahren mit Powervektoren aus der Differenz der vorhergesagten subjektiven Wellenfront und der objektiven Wellenfront.

Schließlich kann eine wie oben dargestellte Korrektur der systematischen Abweichungen zwischen objektiver und subjektiver Refraktion für Objekte im Unendlichen (genannt Fernrefraktion) ebenfalls auf die Nahverordnung, die auch Nahrefraktion genannt wird, angewendet werden.

Die objektive Nahrefraktion liegt im günstigsten Fall als Wellenfront in derselben Entfernung d zum Auge vor, in dem auch die Korrekturen der systematischen Abweichungen der subjektiven und objektiven Fernrefraktion vorliegen. Ist das nicht der Fall, so muss sie nach Stand der Technik in diese Entfernung umgerechnet werden. Dasselbe gilt für die subjektive Nahrefraktion.

Liegt die Nahrefraktion nicht als Wellenfront vor, sondern als Wirkung eines Brillenglases, so ist zu beachten, dass zur Umrechnung nicht die Nahrefraktion selbst propagiert werden darf. Vielmehr muss eine von einem Punkt in der zur Nahrefraktion gehörenden Objektentfernung ausgehende sphärische Wellenfront, welche durch ein gedachtes, die Nahrefraktion enthaltendes, Refraktionsglas gebrochen wurde, im zur Nahrefraktion gehörenden Abstand (sog. Hornhaut-Scheitel-Abstand) zum Auge, propagiert werden.

Die Korrektur der systematischen Abweichungen kann nun auf die so berechnete Wellenfront angewendet werden. Will man wieder eine korrigierte Nahrefraktion erhalten - diesmal aber in der Entfernung $d$ zum Auge - so muss die Differenz zu einer sphärischen Referenzwellenfront berechnet werden, welche ausgehend von einem sich in der Refraktionsentfernung befindenden Punkt in die selbe Entfernung $d$ propagiert wurde.

**Schritt 2** - **Kombination der korrigierten Wellenfronten bzw. der korrigierten Komponenten der Refraktion**

Die Powervektoren der subjektiven und objektiven Wellenfronten $P_{sub}$ und $P_{obj}$ werden nach der Korrektur systematischer Unterschiede gewichtet gemittelt. Von großer Bedeutung sind die Gewichte des sphärischen Äquivalents M, da sich in Abhängigkeit vom Akkommodationsvermögen und speziell bei dessen Einschränkung in Folge des Alterungsprozesses der Augenlinse das Risiko einer zu myopen Refraktion verändert. Die Gewichte der astigmatischen Komponenten, d.h. für J0 und J45, können z.B. für die subjektive Refraktion auf 0,7, die entsprechenden objektiven Komponenten auf 0,3 gesetzt werden.

Gemäß einem Beispiel werden vorteilhafte Gewichte des sphärischen Äquivalents vorgeschlagen, die eine besonders genaue Schätzung der Fehlsichtigkeit des Brillenträgers ermöglichen. Die Motivation für die vorgeschlagenen Gewichte des sphärischen Äquivalents ist aus dem folgenden Gedanken entstanden:

In den Fällen, in denen objektive und subjektive sphärische Äquivalente konsistent sind, sollten beide Datenquellen genutzt werden. Sollten die Messungen nicht konsistent sein, so sollten die Gewichte abhängig vom Risiko einer zu myopen Messung angepasst werden: Je niedriger die Addition ist, desto höher ist das Risiko einer zu myopen Messung (sowohl bei der subjektiven als auch bei der objektiven Refraktion). In diesem Fall wird dem positiveren sphärischen Äquivalent ein höheres Gewicht gegeben. Je höher die Addition, desto geringer ist das Risiko einer zu myopen Messung, so dass eine große Abweichung der subjektiven und objektiven sphärischen Äquivalente wahrscheinlich andere Gründe haben wird. Deshalb wird vorzugsweise in diesen Fällen die subjektive Messung hoch gewichtet, da die refraktionierte Person während der Refraktion bereits ein entsprechendes Glas getestet hat.

Berechnung der Gewichte

Die Gewichte können abhängig von der Differenz der objektiv und subjektiv bestimmten sphärischen Äquivalente,

$$\Delta M = M_{sub} - M_{obj},$$

$$(12)$$

und abhängig vom Akkommodationsvermögen, das aus der Addition bestimmt werden kann,

$$Akk = -(Add + A_1^N),$$

$$(13)$$

berechnet bzw. festgelegt werden.

Dabei sind:

$M_{sub}$: sphärisches Äquivalent aus subjektiver Refraktion
$M_{obj}$: sphärisches Äquivalent aus objektiver Refraktion
$Akk$: aus der Addition errechnetes Akkommodationsvermögen
$Add$: bei der Refraktion gemessene Addition, oder die verordnete Addition
$A_1^N$ : reziproker Objektabstand bei der Additionsbestimmung (negative Vorzeichenkonvention, d.h. $A_1^N < 0$ , z.B. bei einem Objekt in 40 cm Entfernung ist $A_1^N = -2,5 \text{Dpt}$ )

Zur Berechnung der Gewichte $g_{sub}^M(\Delta M, Akk)$ des subjektiv bestimmten sphärischen Äquivalents $M_{sub}$ können zunächst abhängig von $\Delta M$ Hilfsgewichte $g_{sub}^M(\Delta M, Akk_1)$ und $g_{sub}^M(\Delta M, Akk_2)$ bestimmt werden:

$$g_{sub}^M(\Delta M, Akk_i)$$

$$= \begin{cases} g_{sub}^M(\Delta M_{-2}, Akk_i) & \text{für } \Delta M \leq \Delta M_{-2} \\ g_{sub}^M(0, Akk_i) \cdot \dfrac{\Delta M - \Delta M_{-2}}{\Delta M_{-1} - \Delta M_{-2}} + g_{sub}^M(\Delta M_{-2}, Akk_i) \cdot \dfrac{\Delta M_{-1} - \Delta M}{\Delta M_{-1} - \Delta M_{-2}} & \text{für } \Delta M_{-2} < \Delta M < \Delta M_{-1} \\ g_{sub}^M(0, Akk_i) & \text{für } \Delta M_{-1} \leq \Delta M \leq \Delta M_{+1} \\ g_{sub}^M(0, Akk_i) \cdot \dfrac{\Delta M_{+2} - \Delta M}{\Delta M_{+2} - \Delta M_{+1}} + g_{sub}^M(\Delta M_{+2}, Akk_i) \cdot \dfrac{\Delta M - \Delta M_{+1}}{\Delta M_{+2} - \Delta M_{+1}} & \text{für } \Delta M_{+1} < \Delta M < \Delta M_{+2} \\ g_{sub}^M(\Delta M_{+2}, Akk_i) & \text{für } \Delta M_{+2} \leq \Delta M, \end{cases}$$

$$(14)$$

wobei für $i$ 1 bzw. 2 eingesetzt wird.

Das subjektive Gewicht erhält man, indem die Hilfsgewichte im Bereich zwischen $Akk_1$ und $Akk_2$ linear interpoliert werden:

$$g_{sub}^M(\Delta M, Akk)$$

$$= \begin{cases} g_{sub}^M(\Delta M, Akk_1) & \text{für } Akk \leq Akk_1 \\ g_{sub}^M(\Delta M, Akk_1) \cdot \dfrac{Akk_2 - Akk}{Akk_2 - Akk_1} + g_{sub}^M(\Delta M, Akk_2) \cdot \dfrac{Akk - Akk_1}{Akk_2 - Akk_1} & \text{für } Akk_1 < Akk < Akk_2 \\ g_{sub}^M(\Delta M, Akk_2) & \text{für } Akk_2 \leq Akk, \end{cases}$$

$$(15)$$

Die Gewichte des objektiv bestimmten sphärischen Äquivalents können aus den Gewichten des subjektiv bestimmten sphärischen Äquivalents durch $g_{obj}^M(\Delta M, Akk) = 1 - g_{sub}^M(\Delta M, Akk)$ berechnet werden.

Im Folgenden sind Bereiche für die Stützstellen und deren Gewichte angegeben:

$$-1{,}5\text{Dpt} \leq \Delta M_{-2} \leq -0{,}5\text{Dpt}$$

$$-1{,}0\text{Dpt} \leq \Delta M_{-1} \leq -0{,}25\text{Dpt}$$

$$0{,}25\text{Dpt} \leq \Delta M_{+1} \leq 1{,}0\text{Dpt}$$

$$0{,}5\text{Dpt} \leq \Delta M_{+2} \leq 1{,}5\text{Dpt}$$

wobei $\Delta M_{-2} < \Delta M_{-1} < \Delta M_{+1} < \Delta M_{+2}$.

$$0\text{Dpt} \leq Akk_1 \leq 1{,}25\text{Dpt}$$

$$1{,}0\text{Dpt} \leq Akk_2 \leq 2{,}75\text{Dpt}$$

wobei $Akk_1 < Akk_2$.

Die Gewichte an den Stützstellen können aus den folgenden Bereichen gewählt werden:

$$0,8 \leq g_{sub}^{M}(\Delta M_{-2}, Akk_1) \leq 1$$

$$0,3 \leq g_{sub}^{M}(0, Akk_1) \leq 0,7$$

$$0,8 \leq g_{sub}^{M}(\Delta M_{+2}, Akk_1) \leq 1$$

$$0 \leq g_{sub}^{M}(\Delta M_{-2}, Akk_2) \leq 0,5$$

$$0,3 \leq g_{sub}^{M}(0, Akk_2) \leq 0,7$$

$$0,8 \leq g_{sub}^{M}(\Delta M_{+2}, Akk_2) \leq 1$$

Beispiele für die Wahl von Gewichten und Stützstellen:

Beispiel 1:

$$-\Delta M_{-2} = \Delta M_{+2} = 1,0\text{Dpt}$$

$$-\Delta M_{-1} = \Delta M_{+1} = 0,5\text{Dpt}$$

$$Akk_1 = 0\text{Dpt}$$

$$Akk_2 = 1,75\text{Dpt}$$

$$g_{sub}^{M}(\Delta M_{-2}, Akk_1) = 0,95$$

$$g_{sub}^{M}(0, Akk_1) = 0,75$$

$$g_{sub}^{M}(\Delta M_{+2}, Akk_1) = 0,95$$

$$g_{sub}^{M}(\Delta M_{-2}, Akk_2) = 0,5$$

$$g_{sub}^{M}(0, Akk_2) = 0,75$$

$$g_{sub}^{M}(\Delta M_{+2}, Akk_2) = 0,95$$

Beispiel 2:

$$-\Delta M_{-2} = \Delta M_{+2} = 0\text{Dpt}$$

$$-\Delta M_{-1} = \Delta M_{+1} = 0\text{Dpt}$$

$$Akk_1 = 0\text{Dpt}$$

$$Akk_2 = 1,75\text{Dpt}$$

$$g_{sub}^{M}(\Delta M_{-2}, Akk_1) = 0,5$$

$$g_{sub}^{M}(0, Akk_1) = 0,75$$

$$g_{sub}^{M}(\Delta M_{+2}, Akk_1) = 1$$

$$g_{sub}^{M}(\Delta M_{-2}, Akk_2) = 0,75$$

$$g_{sub}^{M}(0, Akk_2) = 0,75$$

$$g_{sub}^{M}(\Delta M_{+2}, Akk_2) = 0,75$$

Beispiel 3:

$$-\Delta M_{-2} = \Delta M_{+2} = 1,5\text{Dpt}$$

$$-\Delta M_{-1} = \Delta M_{+1} = 0,75\text{Dpt}$$

$$Akk_1 = 0,5\text{Dpt}$$

$$Akk_2 = 2,0\text{Dpt}$$

$$g_{sub}^{M}(\Delta M_{-2}, Akk_1) = 1$$

$$g_{sub}^{M}(0, Akk_1) = 0,5$$

$$g_{sub}^{M}(\Delta M_{+2}, Akk_1) = 1$$

$$g_{sub}^{M}(\Delta M_{-2}, Akk_2) = 0$$

$$g_{sub}^{M}(0, Akk_2) = 0,5$$

$$g_{sub}^{M}(\Delta M_{+2}, Akk_2) = 1$$

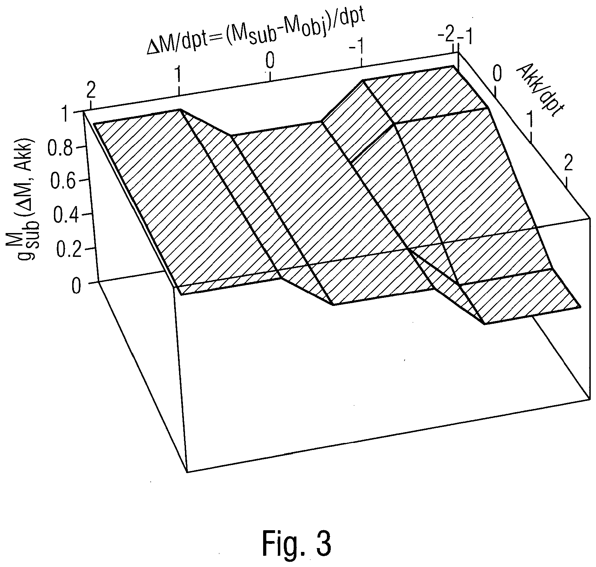

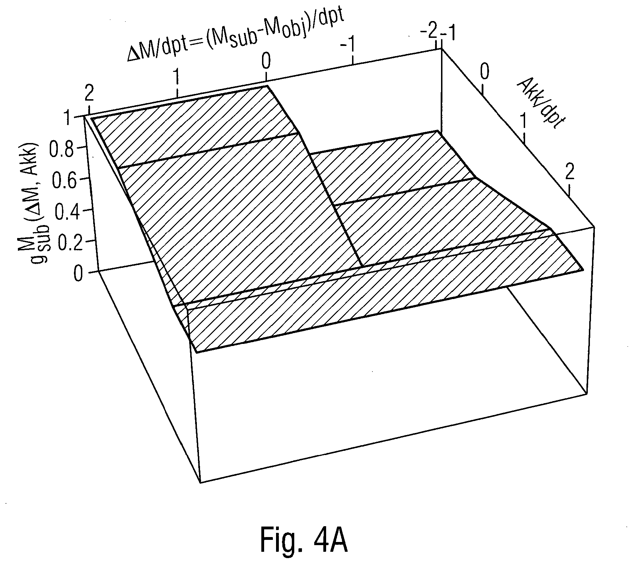

**Figur 3** zeigt die Gewichte des subjektiven sphärischen Äquivalents $g_{sub}^{M}$ gemäß dem Beispiel 1. **Figur 4A** zeigt die Gewichte des subjektiven sphärischen Äquivalents $g_{sub}^{M}$ gemäß dem Beispiel 2. **Figur 4C** und zeigt die Gewichte des subjektiven sphärischen Äquivalents $g_{sub}^{M}$ gemäß dem Beispiel 3. Die in Fig. 3 gezeigten Gewichte sind im Hinblick auf eine Reduzierung der statistischen Messungenauigkeiten der subjektiven und/oder objektiven Messung vorteilhafter als die in Fig. 4A gezeigten Gewichte.

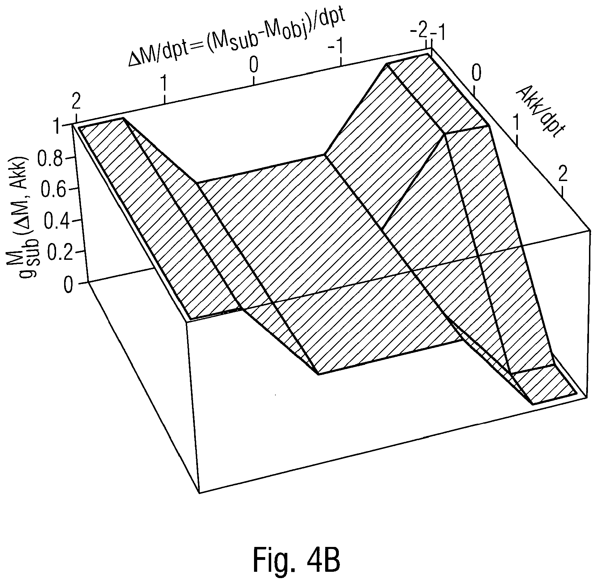

Die Gewichte gemäß dem Beispiel 1 (Fig. 3), dem Beispiel 2 (Fig. 4A) und dem Beispiel 3 (Fig. 4B) hängen von der Addition und dem Unterschied (bzw. der Differenz) der im Schritt 1 korrigierten sphärischen Äquivalente $\Delta M = M_{sub} - M_{obj}$ ab. Die Funktionen bestehen aus mehreren Plateaus konstanten Gewichts, zwischen denen, wie oben beschrieben, linear interpoliert wird.

Ein wesentlicher Unterschied in der Wahl der Gewichte gemäß dem Beispiel 1 im Vergleich zur Wahl der Gewichte gemäß dem Beispiel 2 besteht darin, dass in dem Bereich, in dem $\Delta M$ näherungsweise Normalverteilt ist, hier z.B. im Bereich -0,5Dpt < $\Delta M$ < +0,5Dpt, ein Plateau mit einem Gewicht von z.B. $g_{sub}^{M} = 0,75$ eingeführt wird. In diesen Bereich fallen die meisten Messungen. Wegen der näherungsweise normalverteilten Differenz $\Delta M$ kann davon ausgegangen werden, dass die sphärischen Äquivalente der subjektiven und objektiven Refraktion nicht widersprüchlich sind, sodass das sphärische Äquivalent der objektiven Refraktion ein relativ hohes Gewicht, z.B. 0,25, erhalten kann. Außerhalb dieses Bereichs steigt bei einer hohen Addition das subjektive Gewicht auf einen sehr hohen Wert, z.B. auf 0,95 oder sogar 1,0, unabhängig vom Vorzeichen des Unterschieds $\Delta M$, da hier Akkommodation sehr unwahrscheinlich ist. Bei niedrigen Additionen wird die subjektive Refraktion nur dann sehr hoch gewichtet, wenn sie hyperoper war als die objektive. Ist sie myoper, so wird das subjektive Gewicht auf einen niedrigen Wert reduziert, z.B. auf 0,5.

Die vom Vorzeichen von $\Delta M$ abhängende Änderung der Gewichte ist der zweite grundlegende Unterschied zwischen beiden Gewichtungsarten: im Verfahren gemäß dem ersten Beispiel findet dies bei niedrigen Additionen statt, beim Verfahren gemäß dem zweiten Beispiel ist dies bei hohen Additionen der Fall.

Um Nahverordnungen zu kombinieren, können die korrigierten zur subjektiven und objektiven Refraktion gehörenden Wellenfronten analog zur Fernrefraktion kombiniert werden. Es ist jedoch von Vorteil, aufgrund der größeren Messunsicherheit die objektive Refraktion wie im Stand der Technik beschrieben nur schwach zu gewichten.

Im Folgenden werden anhand eines (relativ kleinen) Datensatzes (Referenzdatensatzes) die Änderungen, die sich gegenüber dem vorbekannten Verfahren für die subjektive, objektive und kombinierte Refraktion ergeben, ausgewertet. Im Folgenden Beispiel wird die subjektive Refraktion nicht geändert und ist deshalb hier nicht dargestellt.

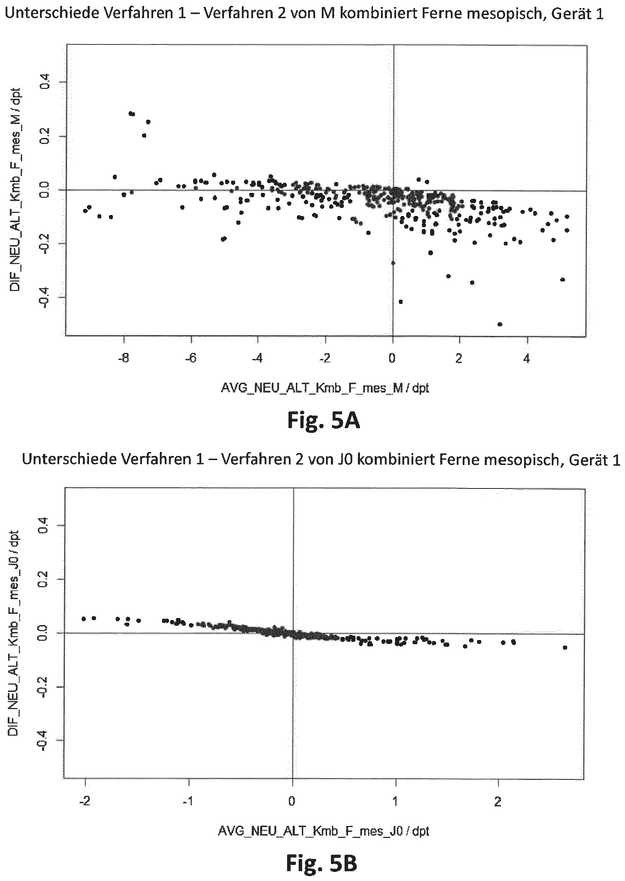

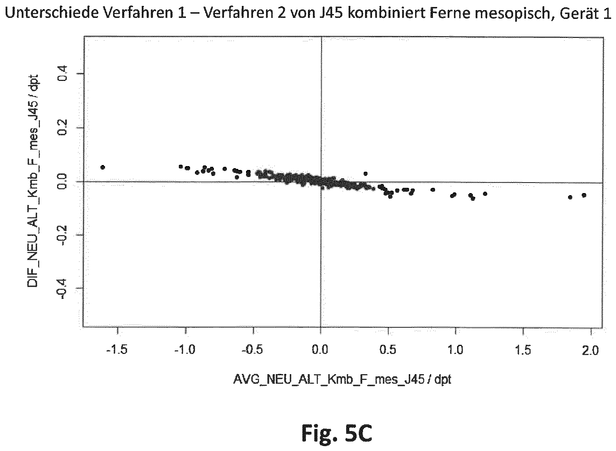

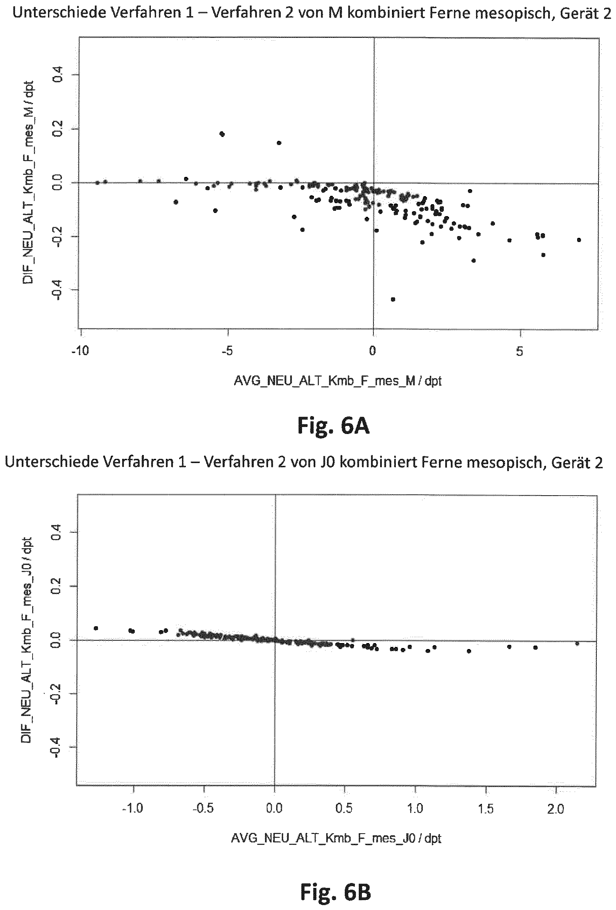

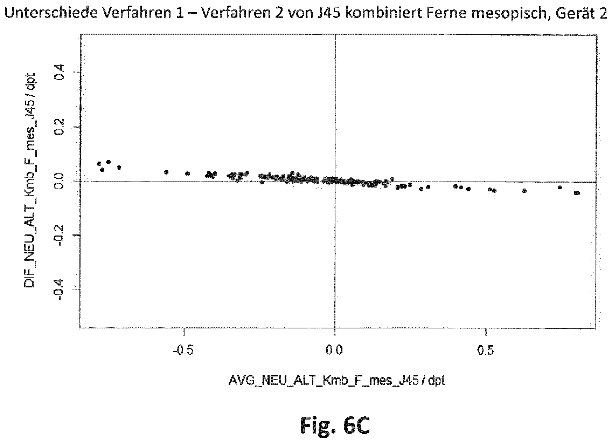

**Figuren 5** und **6** zeigen die Änderung des nach zwei unterschiedlichen Verfahren berechneten Schätzwertes der Fehlsichtigkeit komb_F) ( bei einer, zwischen einer photopischen und einer mesopischen Pupille interpolierten Pupille für zwei unterschiedliche Geräte. Insbesondere zeigen die Figuren 5 und 6 die Differenz der Werte, welche nach einem ersten Verfahren umfassend die oben beschriebenen Schritte 1 und 2 und mit den Gewichten nanch Beispiel 1 erhalten werden und einem zweiten Verfahren, welches ohne Angleich der objektiven an die subjektive Refraktion im Mittel (d.h. ohne Schritt 1, nur Schritt 2) erfolgt und mit den Gewichten nach Beispiel 22. Die objektiven Messwerte werden mit zwei unterschiedlichen Geräten (Aberrometer), Aberrometer 1 und 2, erhalten, wobei Fig. 5 die Ergebnisse für den ersten Aberrometer und Fig. 6 die Ergebnisse für den zweiten Aberrometer zeigt. Figuren 5A und 6A zeigen die Unterschiede des sphärischen Äquivalents M, Figuren 5B und 6B die Unterschiede der Komponente J0 und Figuren 5C und 6C die Unterschiede für die Komponente J45.

Die objektive Refraktion bei einer aus zwei anderen Pupillen geschätzten Pupille (einer photopischen Pupille und einer mesopischen Pupille), zeigt die erwarteten Unterschiede, die sich durch Schritt 1 des Verfahrens ergeben. Die aus subjektiver korrigierter Refraktion und objektiver korrigierter Refraktion kombinierte Refraktion bei der interpolierten Pupille zeigt ebenfalls die erwarteten Änderungen, die hauptsächlich durch den Angleich objektiv an subjektiv entstehen.

Die "Ausreißer" in den Darstellungen sind alle nicht-Presbyope, bei denen das Risiko einer zu myopen Refraktion besteht. Bei ihnen wird die stärker positive Refraktion hoch gewichtet.

Ein Vorgehen, bei hoher Addition pauschal die Refraktion mit mehr Plus stärker zu gewichten, würde bei bereits behobenen systematischen Unterschieden der beiden Refraktionen selbst ohne "Ausreißer", wie z.B. Gerätemyopie, zu einer systematischen Verschiebung der kombinierten Refraktion in Richtung Plus führen. Dies ist unerwünscht, da eine zu hyperope Refraktion nicht durch Akkommodation oder Blicksenkung im Gleitsichtglas kompensiert werden kann. Da Gerätemyopie bei hohen Additionen unwahrscheinlich ist und erst bei niedrigen Additionen auftreten kann, führt das neue Verfahren mit dem Schritt 1) und gegebenenfalls Schritt 2) mit den in Fig. 3 gezeigten Gewichten in diesem Aspekt zu einer besseren kombinierten Refraktion.

Das Beheben der systematischen Fehler der objektiven Refraktion (Schritt 1) zusammen mit einer additions-abhängigen Gewichtung der Refraktion (Schritt 2) ist im Vergleich mit einem alternativen Verfahren, bei dem die systematisch abweichende Refraktion lediglich gering gewichtet, aber nicht verschoben wird, insbesondere deshalb vorteilhafter, weil die mittlere subjektive Refraktion selbst dann mit hoher Genauigkeit aus der objektiven Refraktion berechnet werden kann, wenn systematische Unterschiede zwischen beiden Refraktionsarten auftreten. Die Wahl der Gewichte sollte stattdessen idealerweise anhand der Vertrauenswürdigkeit der bereits korrigierten Refraktionsmethoden erfolgen.

Insgesamt führt das vorgeschlagene Verfahren mit den Schritten 1 und 2 zu einer Abnahme der Reklamationswahrscheinlichkeit bei Brillengläsern, bei denen sowohl subjektive als auch objektive Refraktion in die Berechnung einfließen, insbesondere in den Wirkungsbereichen, in denen Aberrometer systematisch anders als die subjektive Refraktion messen.

Nachfolgend sind werden Beispiele von Serien von Brillengläsern, die mit dem oben beschriebenen Verfahren berechnet und hergestellt werden können.

**Brillenglasserie B1:**

Serie von Brillengläsern zur Korrektur der Fehlsichtigkeit einer Vielzahl von Augen, die mindestens ein erstes Brillenglas A umfasst, welches zumindest in einem Bezugspunkt eine erste Wirkung P_A besitzt, die eine durch zumindest

einen mit einer Messvorrichtung einer ersten Art bestimmten aus mehreren Komponenten bestehenden ersten Messwert P_A1 und
einen mit einer Messvorrichtung einer zweiten Art bestimmten aus mehreren Komponenten bestehenden zweiten Messwert P_A2
charakterisierte Fehlsichtigkeit eines ersten Auges korrigiert,

wobei

der mit einer Messvorrichtung der ersten Art bestimmte erste Messwert P_A1 des ersten Glases und der mit einer Messvorrichtung der zweiten Art bestimmte zweite Messwert P_A2 des ersten Glases sich in zumindest einer Komponente X unterscheiden,
die Komponente X der im Bezugspunkt des ersten Brillenglases vorhandenen ersten Wirkung P_A des ersten Glases näher an der Komponente X desjenigen Messwerts P_A1 oder P_A2 des ersten Glases liegt, dessen Messvorrichtung die geringere Ungenauigkeit bei der Messung der Komponente X besitzt, und wobei
die Komponenten X eine Komponente einer Wellenfrontdarstellung der Fehlsichtigkeit, deren Linearkombination oder daraus abgeleitete Größen sind.

**Brillenglasserie B2:**

Serie von Brillengläsern gemäß Serie B1, wobei ferner
die Komponente X der im Bezugspunkt des ersten Brillenglases vorhandenen ersten Wirkung P_A und die Komponente X des ersten Messwerts des ersten Auges P_A1 nahezu identisch sind, obwohl sich der erste Messwert des ersten Auges P_A1 und der zweite Messwert des ersten Auges P_A2 zumindest in der Komponente X unterscheiden.

**Brillenglasserie B3:**

Serie von Brillengläsern gemäß Serie B1 oder B2, welche

mindestens ein zweites Brillenglas B umfasst, welches zumindest in einem im Vergleich zum ersten Brillenglas identisch bezeichneten Bezugspunkt eine zweite Wirkung P_B besitzt, die eine durch zumindest einen mit einer Messvorrichtung der ersten Art bestimmten aus mehreren Komponenten bestehenden ersten Messwert P_B1 und einen mit einer Messvorrichtung der zweiten Art bestimmten aus mehreren Komponenten bestehenden zweiten Messwert P_B2 charakterisierte Fehlsichtigkeit eines zweiten Auges korrigiert, und
mindestens ein drittes Brillenglas C umfasst, welches zumindest in einem im Vergleich zum ersten Brillenglas identisch bezeichneten Bezugspunkt eine dritte Wirkung P_B besitzt, die eine durch zumindest einen mit einer Messvorrichtung der ersten Art bestimmten aus mehreren Komponenten bestehenden ersten Messwert P_C1 und einen mit einer Messvorrichtung der zweiten Art bestimmten aus mehreren Komponenten bestehenden zweiten Messwert P_C2 charakterisierte Fehlsichtigkeit eines dritten Auges korrigiert, wobei

die mit Messvorrichtungen der ersten Art bestimmten ersten Messwerte P_A1, P_B1 und P_C1 des ersten, zweiten und dritten Auges komponentenweise identisch sind,
die Komponenten X der mit Messvorrichtungen der zweiten Art bestimmten zweiten Messwerte P_A2, P_B2 und P_C2 des ersten, zweiten und dritten Auges sich alle paarweise unterscheiden,
die Komponente X der im Bezugspunkt des ersten Brillenglases vorhandenen ersten Wirkung P_A und
die Komponente X des ersten Messwerts des ersten Auges P_A1 nahezu identisch sind, und wobei
für die Komponenten X der im Bezugspunkt vorhandenen Wirkung des i-ten Brillenglases, X_i, und für die Komponenten X der zweiten Messwerte der i-ten Augen, X_i2, folgende Zusammenhänge gelten:

$$(X\_B - X\_A) / (X\_B2 - X\_A2) \text{ ungleich } (X\_C - X\_A) / (X\_C2 - X\_A2)$$

$$\text{abs}(X\_B2 - X\_A2) < \text{abs}(X\_C2 - X\_A2)$$

$$\text{signum}(X\_B2 - X\_A2) = \text{signum}(X\_C2 - X\_A2).$$

**Brillenglasserie B4:**

Serie von Brillengläsern gemäß Serie B3, wobei:

die ersten, zweiten und dritten Brillengläser Einstärkengläser oder über dieselbe Addition Add verfügende Gleitsichtgläser sind,
wobei Add <= 1,5dpt, und wobei
für die Komponenten X der im Bezugspunkt vorhandenen Wirkung des i-ten Brillenglases, X_i, und für die Komponenten X der zweiten Messwerte der i-ten Augen, X_i2, folgende Zusammenhänge gelten:

$$(X\_B - X\_A) / (X\_B2 - X\_A2) < (X\_C - X\_A) / (X\_C2 - X\_A2) \quad \text{falls}$$
$$X\_B2 - X\_A2 > 0, \quad X\_C2 - X\_A2 > 0,$$

und

$$(X\_B - X\_A) / (X\_B2 - X\_A2) > (X\_C - X\_A) / (X\_C2 - X\_A2) \quad \text{falls}$$
$$X\_B2 - X\_A2 < 0, \quad X\_C2 - X\_A2 < 0.$$

**Brillenglasserie B5:**

Serie von Brillengläsern gemäß Serie B3, wobei

die ersten, zweiten und dritten Brillengläser über dieselbe Addition Add verfügende Gleitsichtgläser sind,
wobei Add >= 2dpt, und wobei
für die Komponenten X der im Bezugspunkt vorhandenen Wirkung des i-ten Brillenglases, X_i, und für die Komponenten X der zweiten Messwerte der i-ten Augen, X_i2, folgende Zusammenhänge gelten:

$$(X\_B - X\_A) / (X\_B2 - X\_A2) > (X\_C - X\_A) / (X\_C2 - X\_A2) \quad \text{falls}$$
$$X\_B2 - X\_A2 > 0, \quad X\_C2 - X\_A2 > 0,$$

uns

$$(X\_B - X\_A) / (X\_B2 - X\_A2) > (X\_C - X\_A) / (X\_C2 - X\_A2) \quad \text{falls}$$
$$X\_B2 - X\_A2 < 0, \quad X\_C2 - X\_A2 < 0.$$

**Brillenglasserie B6:**

Serie von Brillengläsern gemäß einer der Serien B1 bis B5, wobei die Messvorrichtung der ersten Art zur subjektiven Refraktion verwendet werden kann.

**Brillenglasserie B7:**

Serie von Brillengläsern gemäß einer der Serien B1 bis B6, wobei die Messvorrichtung der zweiten Art zur Bestimmung der objektiven Refraktion verwendet werden kann.

**Brillenglasserie B8:**

Serie von Brillengläsern gemäß einer der Serien B1 bis B6, welche:

mindestens ein viertes Brillenglas D umfasst, welches zumindest in einem im Vergleich zum ersten Brillenglas identisch bezeichneten Bezugspunkt eine vierte Wirkung P_D besitzt, die eine durch zumindest einen mit einer Messvorrichtung der ersten Art bestimmten aus mehreren Komponenten bestehenden ersten Messwert P_D1 und einen mit einer Messvorrichtung der zweiten Art bestimmten aus mehreren Komponenten bestehenden zweiten Messwert P_D2 charakterisierte Fehlsichtigkeit eines vierten Auges korrigiert, und

mindestens ein fünftes Brillenglas E umfasst, welches zumindest in einem Vergleich zum ersten Brillenglas identisch bezeichneten Bezugspunkt eine fünfte Wirkung P_E besitzt, die eine durch zumindest mit einer Messvorrichtung der ersten Art bestimmten aus mehreren Komponenten bestehenden ersten Messwert P_E1 und einen mit einer Messvorrichtung der zweiten Art bestimmten aus mehreren Komponenten bestehenden zweiten Messwert P_E2 charakterisierte Fehlsichtigkeit eines vierten Auges korrigiert, und wobei

die mit Messvorrichtungen der ersten Art bestimmten ersten Messwerte P_A1, P_D1 und P_E1 des ersten, vierten und fünften Auges komponentenweise identisch sind,
die Komponenten X der mit Messvorrichtungen der zweiten Art bestimmten zweiten Messwerte P_A2, P_D2 und P_E2 des ersten, vierten und fünften Auges sich alle paarweise unterscheiden,
die Komponente X der im Bezugspunkt des ersten Brillenglases vorhandenen ersten Wirkung P_A und die Komponente X des ersten Messwerts des ersten Auges P_A1 nahezu identisch sind, und wobei
für die Komponenten X der im Bezugspunkt vorhandenen Wirkung des i-ten Brillenglases, X_i, und für die Komponenten X der zweiten Messwerte der i-ten Augen, X_i2, folgende Zusammenhänge gelten:
X_D2 - X_A2 > 0, X_E2 - X_A2 < 0, X_D - X_A > 0 und X_E - X_A < 0.

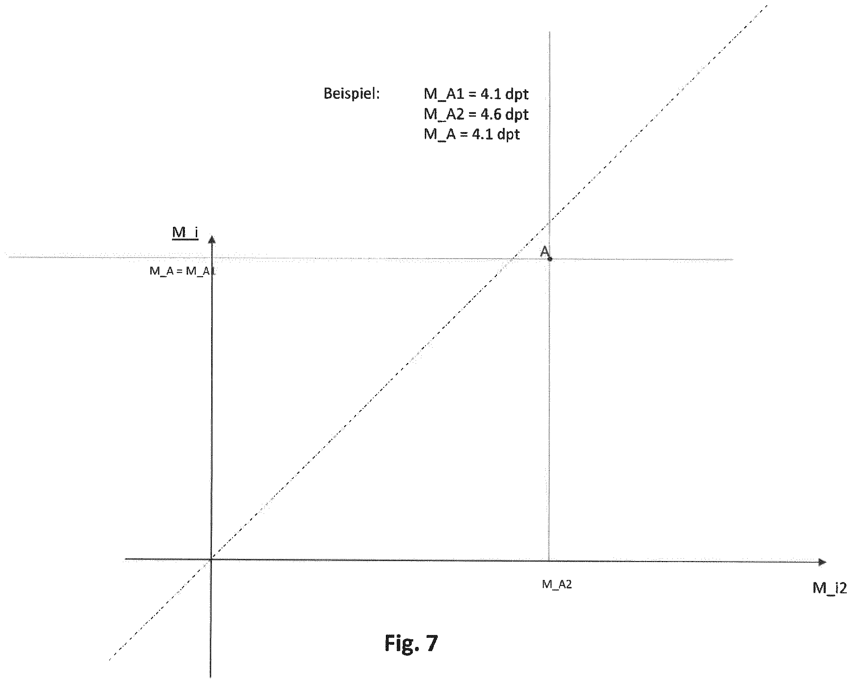

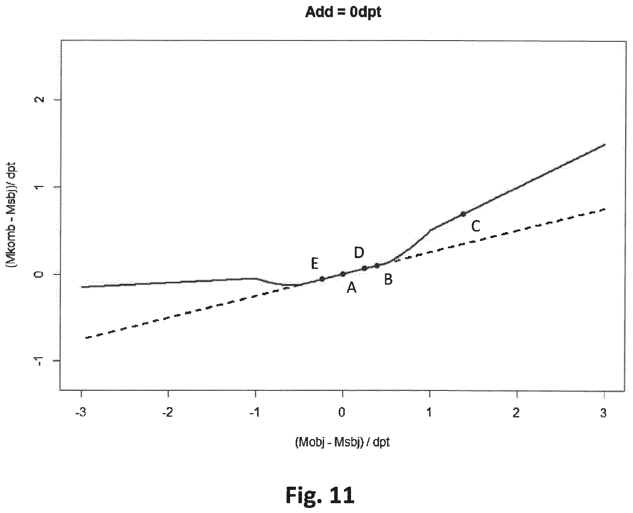

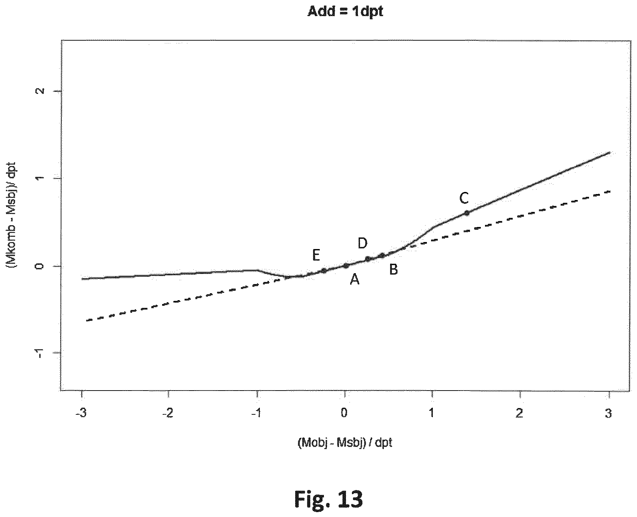

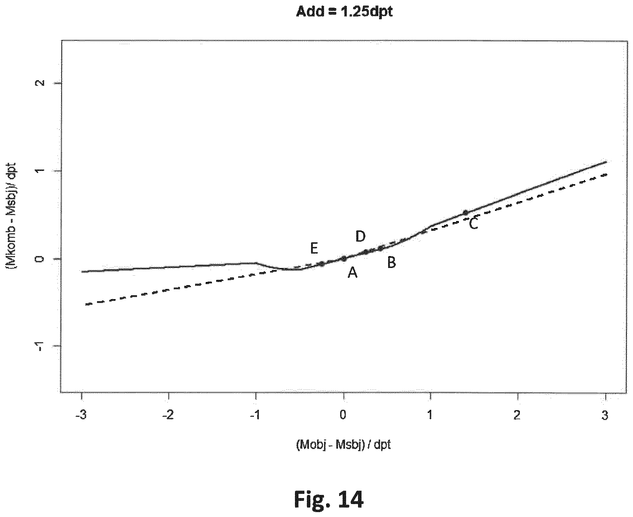

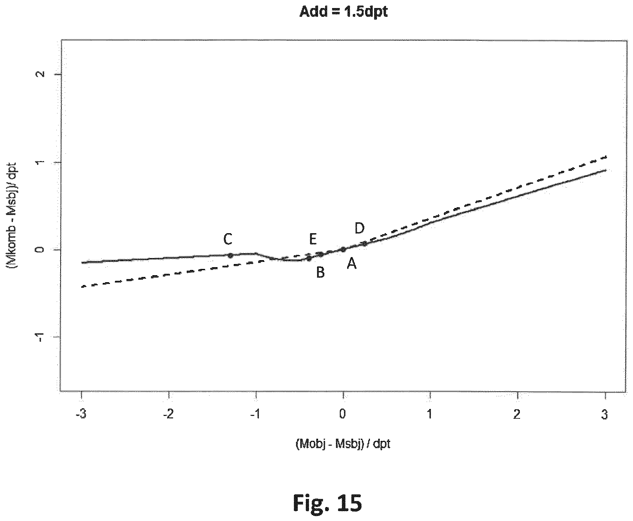

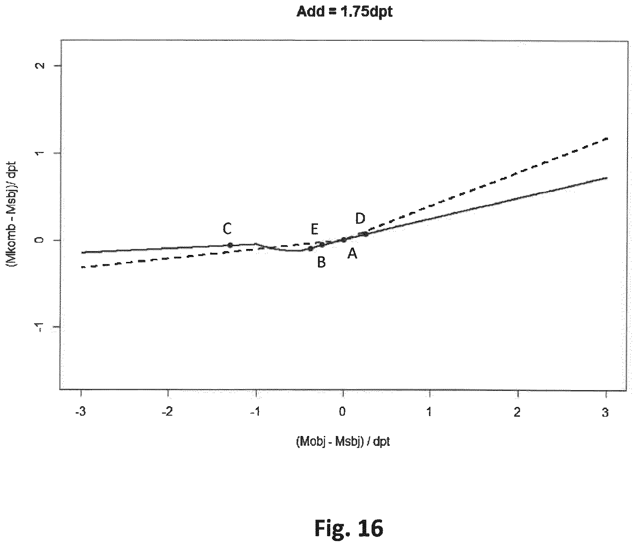

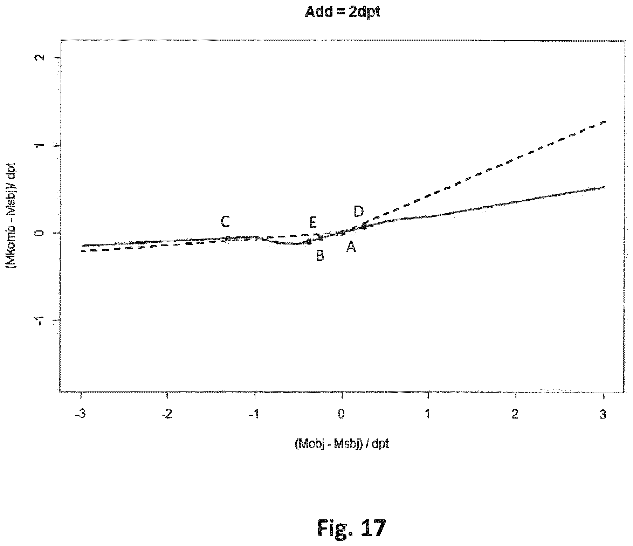

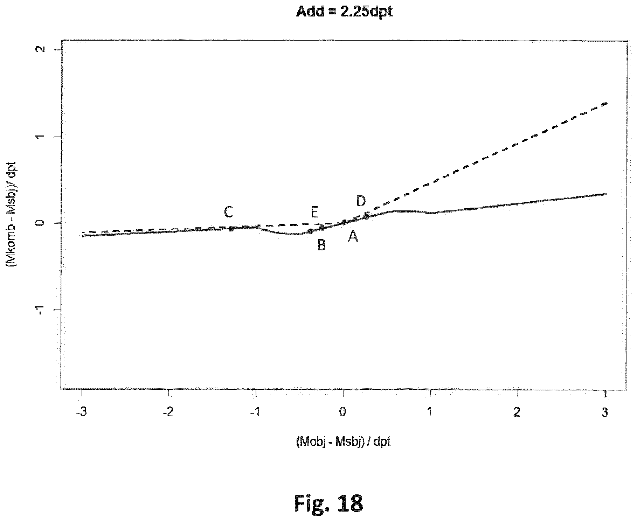

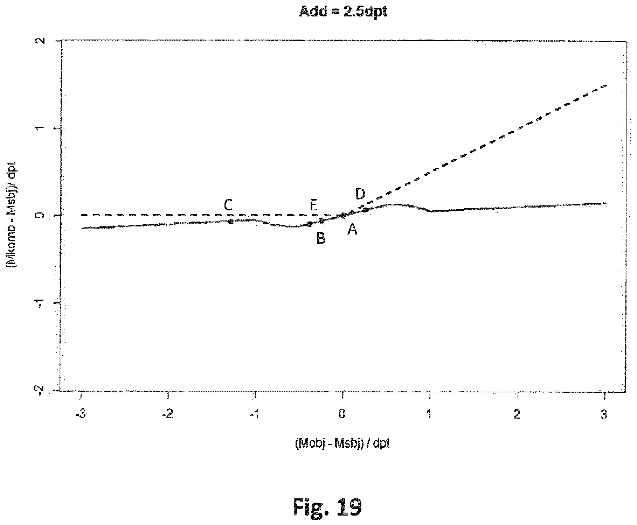

**Figuren 7** bis **10** zeigen einzelne repräsentative Brillengläser der obigen Serien von Brillengläsern. Die gezeigten Brillengläser weisen ausgewählte Eigenschaften auf, die es erlauben, Eigenschaften der von $\Delta M$ abhängigen Gewichte aus Figuren 11 bis 20 anhand von 1, 3 oder 5 Gläsern einer Serie festzustellen, unabhängig davon, ob eine Korrektur der systematischen Fehler durchgeführt wurde oder nicht. Die auf den durchgezogenen bzw. gestrichelten Linien in den Detailfiguren liegenden Brillengläser besitzen dasselbe subjektive sphärische Äquivalent (auf der dargestellten Linie beträgt das subjektive sphärische Äquivalent M_A1 = M_A = 4,1 Dpt). Die durchgezogenen bzw. gestrichelten Linien beziehen sich auf Stützstellen und Gewichte aus Beispiel 1 bzw. 2.
**Figur 7** bezieht sich auf ein Brillenglas A aus der Brillenglasserie B2, das mit einem Verfahren umfassend Schritt 1 berechnet wurde. Das in Fig. 7 gezeigte Brillenglas A weist im Bezugspunkt das subjektiv gemessene sphärische Äquivalent als Wirkung auf (d.h. M_A = MA_1 = 4,1 Dpt), obwohl sich das objektiv gemessene sphärische Äquivalent (M_A2 = 4,6 Dpt) wesentlich von dem subjektiv gemessene sphärische Äquivalent (M_A1 = 4,1 Dpt) unterscheidet. Das objektiv gemessene sphärische Äquivalent wurde dennoch bei der Berechnung berücksichtigt.

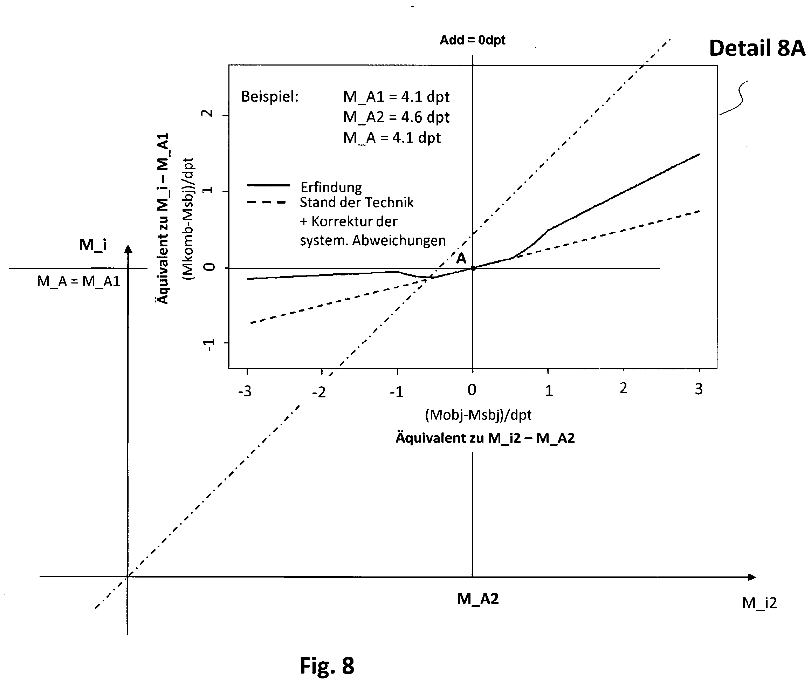

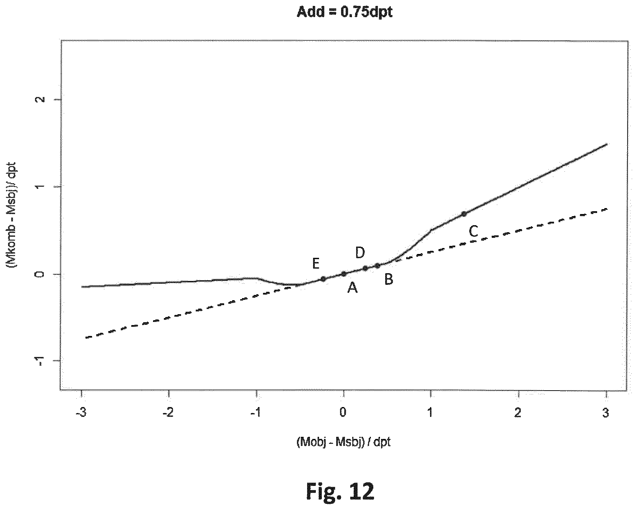

**Figur 8** bezieht sich auf ein anderes Brillenglas A aus einer Brillenglasserie B2, das mit einem Verfahren umfassend die Schritte 1 und 2 berechnet wurde. Das in Fig. 8A gezeigte Brillenglas weist ähnliche Eigenschaften, wie das in Fig. 7 gezeigte Brillenglas auf. Das Detail 8A entspricht Figur 11 und 12.

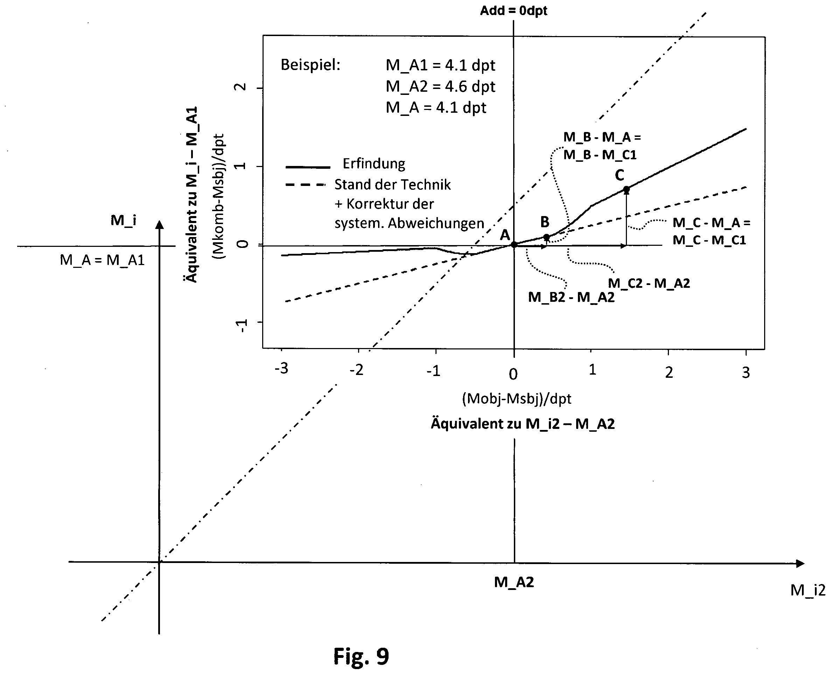

**Figur 9** bezieht sich auf 3 Brillengläser A, B, C aus der Brillenglasserie B3, welche mit einem Verfahren umfassend die Schritte 1 und 2 berechnet wurden. Kennzeichnend ist hierbei, dass alle Gläser dasselbe subjektive sphärische Äquivalent besitzen, und dass die iso-Linie gleichen sphärischen Äquivalents zumindest in einem der Intervalle Mobj < M_A2 und Mobj > M_A2 unterschiedliche Steigungen besitzt - dies wird durch die Zusammenhänge der gemessenen und im Bezugspunkt vorhandenen sphärischen Äquivalente der Gläser A, B und C ausgedrückt.

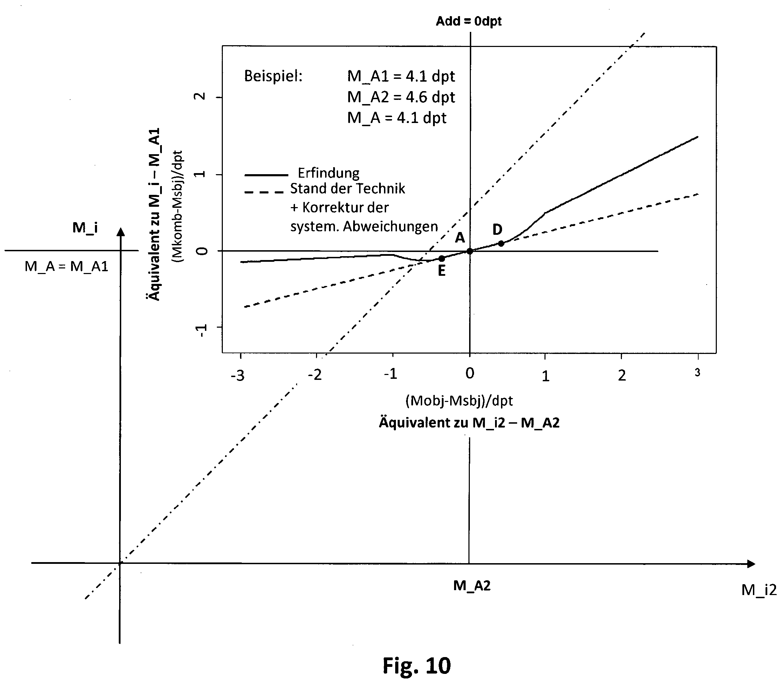

**Figur 10** bezieht sich auf Brillengläser A, D und E aus der Brillenglasserie B8. Kennzeichnend hierbei ist die beidseitig von M_A2 identische Steigung der iso-Linie gleichen sphärischen Äquivalents, die durch die Zusammenhänge der gemessenen und im Bezugspunkt vorhandenen sphärischen Äquivalente der Gläser A, D und E ausgedrückt.
**Figuren 11** bis **19** zeigen die Differenz (Mkomb - Msbj) zwischen einem Schätzwert (Mkomb) des sphärischen Äquivalents und einem gemessenen subjektiven sphärischen Äquivalent (Msbj) als Funktion der Differenz zwischen einem gemessenen objektiven sphärischen Äquivalent (Mobj) und einem gemessenen subjektiven sphärischen Äquivalent für unterschiedliche Additionen Add. "Mkomb" bezeichnet das kombinierte sphärische Äquivalent, d.h. ein nach einem beispielhaften Verfahren umfassend Schritte 1 und/oder 2 berechneten Schätzwert des sphärischen Äquivalents. Auf der x-Achse ist die Differenz des objektiven sphärischen Äquivalents Mobj (z.B. M_B2 oder M_C2) für ein Brillenglas (z.B. ein Brillenglas B oder C) minus dem objektiven sphärischen Äquivalent Mobj=Msbj, bei dem das kombinierte sphärische Äquivalent gleich dem subjektiven sphärischen Äquivalent ist (z.B. M_B2 - M_A2 bzw. MC_2 - M_A2). Figuren 11 bis 19 beziehen sich auf Additionen, die bei der Standard-Objektentfernung von 40cm (entspricht $A_1^N = -2,5\,\mathrm{Dpt}$) bestimmt wurden.

Die durchgezogene bzw. gestichelte Linien zeigen die Fälle, bei denen das kombinierte sphärische Äquivalent Mkomb nach einem beispielhaften Verfahren umfassend Schritt 2 mit Stützpunkten und Gewichten aus Beispiel 1 (durchgezogen) bzw. Beispiel 2 (gestrichelt) erhalten wurde. Das kombinierte sphärische Äquivalent "Mkomb" stellt somit den Schätzwert der Fehlsichtigkeit nach einem beispielhaften Verfahren umfassend Schritt 2 oder die Schritte 1 und 2 dar.

**Patentansprüche**

1.  Computerimplementiertes Verfahren zum Bestimmen der Fehlsichtigkeit eines Auges eines Brillenträgers, umfassend:

    Bereitstellen von Messwerten aus einer ersten und einer zweiten Messung der Fehlsichtigkeit des Auges des Brillenträgers;

    Berechnen eines Schätzwerts für die Fehlsichtigkeit des Auges des Brillenträgers anhand der Messwerte aus der ersten und der zweiten Messung, wobei bei der Berechnung des Schätzwerts der Fehlsichtigkeit Messungenauigkeiten der ersten und der zweiten Messungen der Fehlsichtigkeit berücksichtigt werden, wobei die Messungenauigkeiten eine systematische Abweichung zwischen den Messwerten aus der ersten Messung und den Messwerten aus der zweiten Messung umfassen; und wobei das Verfahren ferner umfasst:

    Bestimmen der Messungenauigkeiten der ersten und der zweiten Messungen mittels statistischer Analyse eines Datensatzes mit einer Vielzahl von Referenzmesswerten, welche eine erste Messung und eine zweite Messung der Fehlsichtigkeit der Augen verschiedener Brillenträger beinhalten, wobei das Bestimmen der Messungenauigkeiten der ersten Messung und der zweiten Messungen umfasst:

      Festlegen eines Modells für die Messwerte der zweiten Messung als eine Summe eines vorhergesagten Messwerts und einer Zufallsvariable, wobei der vorhergesagte Messwert modelliert ist als eine parametrische Funktion des Messwerts der ersten Messung und optional eines Teils des Messwerts der zweiten Messung;

      Ermitteln der Parameter der parametrischen Funktion durch Anpassen des Modells an die im Datensatz enthaltenen Referenzmessungen unter Maximieren der Wahrscheinlichkeitsverteilung der Zufallsvariablen im Parameterraum des Modells;

      Bestimmen der systematischen Abweichung der ersten Messung von der zweiten Messung anhand der vorhergesagten Messung.

2.  Verfahren nach Anspruch 1, wobei die Messungenauigkeiten ferner eine statistische Abweichung zwischen den Messwerten aus der ersten Messung und den Messwerten aus der zweiten Messung umfassen.

3.  Verfahren nach Anspruch 1 oder 2, wobei

    die erste Messung der Fehlsichtigkeit des Auges eine objektive Refraktion ist; und/oder die zweite Messung der Fehlsichtigkeit des Auges eine subjektive Refraktion ist.

4.  Verfahren nach Anspruch 3, wobei die vorhergesagte Messung eine vorhergesagte Refraktion ist, welche modelliert wird durch die folgenden parametrischen Funktionen:

    Modell 1:

$$M_{pred}\left(\widetilde{M}_{obj}, \widetilde{J0}_{obj}, \widetilde{J45}_{obj}\right) = \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj}$$

$$J0_{pred}\left(\widetilde{M}_{obj}, \widetilde{J0}_{obj}, \widetilde{J45}_{obj}\right) = a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj}$$

$$J45_{pred}\left(\widetilde{M}_{obj}, \widetilde{J0}_{obj}, \widetilde{J45}_{obj}\right) = a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i}$$

oder
Modell 2:

$$M_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj} + b_{J0,1}^{M} \widetilde{J0}_{sub} + b_{J45,1}^{M} \widetilde{J45}_{sub}$$

$$J0_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj} + b_{M,1}^{J0} \widetilde{M}_{sub} + b_{J45,1}^{J0} \widetilde{J45}_{sub}$$

$$J45_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i} + b_{M,1}^{J45} \widetilde{M}_{sub} + b_{J0,1}^{J45} \widetilde{J0}_{sub}$$

wobei:

$(M_{pred}, J0_{pred}, J45_{pred})$ den Powervektor der vorhergesagten Refraktion bezeichnet;(

$\widetilde{M}_{obj}, \widetilde{J0}_{obj}, \widetilde{J45}_{obj}$ ) den Powervektor der Messwerte aus der objektiven Refraktion bezeichnet;(

$\widetilde{M}_{sub}, \widetilde{J0}_{sub}, \widetilde{J45}_{sub}$ ) den Powervektor der Messwerte aus der subjektiven Refraktion bezeichnet;

$a_{X,i}^{Y}$ und $b_{Z,i}^{Y}$ die Parameter der jeweiligen parametrischen Funktion bezeichnen,
$Y$ für eine Powervektor-Komponente des Powervektors der vorhergesagten Refraktion steht;
$X$ für eine Powervektor-Komponente des Powervektors der gemessenen objektiven Refraktion steht; und
$\underline{Z}$ für eine Powervektor-Komponente des Powervektors der gemessenen subjektiven Refraktion steht.

5. Verfahren nach einem der vorangegangenen Ansprüche, wobei die Berechnung des Schätzwertes der Fehlsichtigkeit des Auges ein Bilden eines gewichteten Mittelwertes der Messwerte aus der ersten Messung und der zweiten Messung umfasst, wobei die erste Messung mit ersten Gewichten und die zweite Messung mit zweiten Gewichten gewichtet wird, wobei optional unter der ersten Messung und der zweiten Messung diejenige Messung mit der geringeren Messungenauigkeit mit höheren Gewichten gewichtet wird.

6. Verfahren nach Anspruch 5 wenn bezogen auf Anspruch 3, wobei die Gewichte abhängig von den Messwerten der Fehlsichtigkeit sind.

7. Verfahren nach Anspruch 6, wobei die Messwerte eine Addition und/oder ein sphärisches Äquivalent umfassen und die Gewichte abhängig von der Addition und/oder der Differenz zwischen dem Messwert des sphärischen Äquivalents aus der ersten Messung und dem Messwert des sphärischen Äquivalents aus der zweiten Messung sind.

8. Verfahren nach einem der vorangegangenen Ansprüche, wobei:

die Messungenauigkeiten bzw. Messabweichungen der ersten Messung und der zweiten Messung für den Objektabstand Unendlich bestimmt werden; und/oder
die Messungenauigkeiten bzw. Messabweichungen der ersten Messung und der zweiten Messung separat für verschiedene Geräte bestimmt werden;
die Messungenauigkeiten bzw. Messabweichungen der ersten Messung und der zweiten Messung bei einer für alle Daten identischen Entfernung zum Auge bestimmt werden.

9. Computerprogrammprodukt welches, wenn es in den Speicher eines Computers geladen und auf diesem ausgeführt wird, bewirkt, dass der Computer ein Verfahren nach einem der Ansprüche 1 bis 8 durchführt.

10. Vorrichtung zum Bestimmen der Fehlsichtigkeit eines Auges eines Brillenträgers mit einer Rechenvorrichtung, welche ausgebildet ist, das Verfahren nach einem der Ansprüche 1 bis 8 durchzuführen.

11. Verfahren zum Herstellen eines Brillenglases, umfassend:

Bestimmen der Fehlsichtigkeit eines Auges des Brillenträgers nach dem Verfahren gemäß einem der Ansprüche 1 bis 8;
Festlegen der Sollwirkung anhand der bestimmten Fehlsichtigkeit, so dass die Sollwirkung des Brillenglases die bestimmte Fehlsichtigkeit in zumindest einem Bezugspunkt zumindest teilweise korrigiert; und
Fertigen des Brillenglases so, dass die Sollwirkung in dem zumindest einen vorgegebenen Bezugspunkt des Brillenglases erreicht wird.

12. Vorrichtung zum Herstellen eines Brillenglases umfassend;

eine Vorrichtung zum Bestimmen der Fehlsichtigkeit eines Auges eines Brillenträgers nach Anspruch 10;
eine Vorrichtung zum Festlegen der Sollwirkung in einem Bezugspunkt des Brillenglases anhand der bestimmten Fehlsichtigkeit, und
eine Fertigungsvorrichtung zum Fertigen des Brillenglases, so dass die Sollwirkung in dem zumindest einen Bezugspunkt des Brillenglases, vorzugsweise in einer vorgegebenen Gebrauchsstellung des Brillenglases, erreicht wird.

13. Computerimplementiertes Verfahren zum Bestellen von Brillengläsern umfassend:

Bereitstellen von Messwerten aus einer ersten Messung und einer zweiten Messung der Fehlsichtigkeit des Auges des Brillenträgers;
Berechnen eines Schätzwerts für die Fehlsichtigkeit des Auges des Brillenträgers nach dem Verfahren gemäß einem der Ansprüche 1 bis 8;.

14. Vorrichtung zum Bestellen von Brillengläsern umfassend:

eine Vorrichtung zum Bereitstellen von Messwerten aus einer ersten Messung und einer zweiten Messung der Fehlsichtigkeit des Auges des Brillenträgers, und
eine Rechenvorrichtung welche ausgebildet ist, einen Schätzwert für die Fehlsichtigkeit des Auges des Brillenträgers anhand der Messwerte aus der ersten Messung und der zweiten Messung nach dem Verfahren gemäß einem der Ansprüche 1 bis 8 zu berechnen.

**Claims**

1. A computer-implemented method for determining the refractive error of an eye of a wearer of spectacles, comprising:

   providing measured values from a first and a second measurement of the refractive error of the eye of the spectacle wearer;
   calculating an estimate of the refractive error of the eye of the eyeglass wearer from the measurements from the first and second measurements, the calculation of the estimate of the refractive error taking into account measurement inaccuracies of the first and second refractive error measurements, the measurement inaccuracies comprising a systematic deviation between the measurements from the first measurement and the measurements from the second measurement; and
   the method further comprising
   determining the measurement inaccuracies of the first and second measurements by statistical analysis of a data set comprising a plurality of reference measurements including a first measurement and a second measurement of the refractive error of the eyes of different spectacle wearers, wherein determining the measurement inaccuracies of the first measurement and the second measurements comprises:

   establishing a model for the measurement values of the second measurement as a sum of a predicted measurement value and a random variable, wherein the predicted measurement value is modelled as a parametric function of the measurement value of the first measurement and optionally a portion of the measurement value of the second measurement;
   determining the parameters of the parametric function by fitting the model to the reference measurements in the data set while maximising the probability distribution of the random variables in the parameter space of the model;
   determining a systematic deviation of the first measurement from the second measurement using the predicted measurement.

2. The method of claim 1, wherein the measurement uncertainties comprise a statistical and/or a systematic deviation between the measurement values from the first measurement and the measurement values from the second measurement.

3. The method according to claim 1 or 2, wherein

   the first measurement of the refractive error of the eye is an objective refraction; and/or
   the second measurement of the refractive error of the eye is a subjective refraction.

4. The method of claim 3, wherein the predicted measurement is a predicted refraction modelled by the following parametric functions:

   model 1:

$$M_{pred}\left(\widetilde{M}_{obj}, \widetilde{J0}_{obj}, \widetilde{J45}_{obj}\right) = \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj}$$

$$J0_{pred}\left(\widetilde{M}_{obj}, \widetilde{J0}_{obj}, \widetilde{J45}_{obj}\right) = a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj}$$

$$J45_{pred}\left(\widetilde{M}_{obj}, \widetilde{J0}_{obj}, \widetilde{J45}_{obj}\right) = a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i}$$

   or
   model 2:

$$M_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj} + b_{J0,1}^{M} \widetilde{J0}_{sub} + b_{J45,1}^{M} \widetilde{J45}_{sub}$$

$$J0_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj} + b_{M,1}^{J0} \widetilde{M}_{sub} + b_{J45,1}^{J0} \widetilde{J45}_{sub}$$

$$J45_{pred}\left(\widetilde{M}_{obj}, \widetilde{J0}_{obj}, \widetilde{J45}_{obj}, \widetilde{J0}_{sub}, \widetilde{J45}_{sub}\right)$$

$$= a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i} + b_{M,1}^{J45} \widetilde{M}_{sub} + b_{J0,1}^{J45} \widetilde{J0}_{sub}$$

wherein
$(M_{pred}, J0_{pred}, J45_{pred})$ denotes the power vector of the predicted refraction;(

$\widetilde{M}_{obj}, \widetilde{J0}_{obj}, \widetilde{J45}_{obj}$ ) denotes the power vector of the measured values from the objective refraction;(

$\widetilde{M}_{sub}, \widetilde{J0}_{sub}, \widetilde{J45}_{sut}$ ) denotes the power vector of the measured values from the subjective refraction;

$a_{X,i}^{Y}$ and $b_{Z,i}^{Y}$ denote the parameters of the respective parametric function,
Y stands for a power vector component of the power vector of the predicted refraction;
X is a power vector component of the power vector of the measured objective refraction; and
$\underline{Z}$ represents a power vector component of the power vector of the measured subjective refraction.

5. The method according to any one of the preceding claims, wherein calculating the refractive error estimate of the eye comprises forming a weighted average of the readings from the first measurement and the second measurement, wherein the first measurement is weighted with first weights and the second measurement is weighted with second weights, optionally wherein among the first measurement and the second measurement, the measurement with the lower measurement inaccuracy is weighted with higher weights.

6. The method according to claim 5 when referred to claim 3, wherein the weights are dependent on the measurement values of the refractive error.

7. The method according to claim 6, wherein the measured values comprise an addition and/or a spherical equivalent and the weights are dependent on the addition and/or the difference between the measured value of the spherical equivalent from the first measurement and the measured value of the spherical equivalent from the second measurement.

8. The method according to any one of the preceding claims, wherein:

   the measurement inaccuracies or measurement deviations of the first measurement and the second measurement are determined for the object distance infinity; and/or
   the measurement accuracies or measurement deviations of the first measurement and the second measurement are determined separately for different devices;
   the measurement accuracies or measurement errors of the first measurement and the second measurement are determined at an identical distance to the eye for all data.

9. A computer program product which, when loaded into and executed on the memory of a computer, causes the computer to perform a method according to any one of claims 1 to 8.

**10.** Apparatus for determining the refractive error of an eye of a wearer of spectacles, comprising a computing device adapted to perform the method of any one of claims 1 to 8.

**11.** A method of manufacturing a spectacle lens comprising:

Determining the refractive error of an eye of a spectacle wearer according to the method according to any one of claims 1 to 8;

determining the target power based on the determined refractive error such that the target power of the spectacle lens at least partially corrects the determined refractive error in at least one reference point; and

manufacturing the spectacle lens in such a way that the desired effect is achieved in the at least one predetermined reference point of the spectacle lens.

**12.** A device for manufacturing a spectacle lens comprising;

a device for determining the refractive error of an eye of a spectacle wearer according to claim 10;

a device for determining the desired effect in a reference point of the spectacle lens on the basis of the determined refractive error, and

a manufacturing device for manufacturing the spectacle lens so that the desired effect is achieved in the at least one reference point of the spectacle lens, preferably in a predetermined position of use of the spectacle lens.

**13.** A computer-implemented method for ordering spectacle lenses comprising:

Providing measured values from a first measurement and a second measurement of the refractive error of the eye of the wearer of the spectacles;

Calculating an estimate of the refractive error of the eyeglass wearer's eye according to the method of any one of claims 1 to 8.

**14.** A device for ordering spectacle lenses comprising:

a device for providing measured values from a first measurement and a second measurement of the refractive error of the eye of the spectacle wearer, and

a calculating device which is designed to calculate an estimated value for the refractive error of the eye of the spectacle wearer on the basis of the measured values from the first measurement and the second measurement according to the method according to one of claims 1 to 8.

**Revendications**

**1.** Procédé mis en oeuvre par ordinateur pour déterminer l'amétropie d'un oeil d'un porteur de lunettes, comprenant :

mise à disposition de valeurs de mesure provenant d'une première et d'une deuxième mesure de l'amétropie de l'œil du porteur de lunettes ;

calculer une estimation de l'amétropie de l'œil du porteur de lunettes à partir des valeurs de mesure provenant des première et deuxième mesures, le calcul de l'estimation de l'amétropie prenant en compte les incertitudes de mesure des première et deuxième mesures de l'amétropie, les incertitudes de mesure comprenant un écart systématique entre les valeurs de mesure provenant de la première mesure et les valeurs de mesure provenant de la deuxième mesure ; et

le procédé comprenant en outre :

déterminer les incertitudes de mesure des première et deuxième mesures par analyse statistique d'un ensemble de données comprenant une pluralité de valeurs de mesure de référence qui comprennent une première mesure et une deuxième mesure de l'amétropie des yeux de différents porteurs de lunettes, la détermination des incertitudes de mesure de la première mesure et des deuxièmes mesures comprenant :

définir un modèle pour les valeurs de mesure de la deuxième mesure comme une somme d'une valeur de mesure prédite et d'une variable aléatoire, la valeur de mesure prédite étant modélisée comme une fonction paramétrique de la valeur de mesure de la première mesure et, éventuellement, d'une partie de la valeur de mesure de la deuxième mesure ;

déterminer les paramètres de la fonction paramétrique en ajustant le modèle aux mesures de référence contenues dans l'ensemble de données, en maximisant la distribution de probabilité des variables aléatoires dans l'espace des paramètres du modèle ;
déterminer un écart systématique entre la première mesure et la deuxième mesure à partir de la mesure prédite.

**2.** Procédé selon la revendication 1, dans lequel les incertitudes de mesure comprennent un écart statistique et/ou un écart systématique entre les valeurs de mesure provenant de la première mesure et les valeurs de mesure provenant de la deuxième mesure.

**3.** Procédé selon la revendication 1 ou 2, dans lequel

la première mesure de l'amétropie de l'œil est une réfraction objective ; et/ou
la deuxième mesure de l'amétropie de l'œil est une réfraction subjective.

**4.** Procédé selon la revendication 3, dans lequel la mesure prédite est une réfraction prédite qui est modélisée par les fonctions paramétriques suivantes :

modèle 1:

$$M_{pred}\left(\widetilde{M}_{obj}, \widetilde{J0}_{obj}, \widetilde{J45}_{obj}\right) = \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj}$$

$$J0_{pred}\left(\widetilde{M}_{obj}, \widetilde{J0}_{obj}, \widetilde{J45}_{obj}\right) = a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj}$$

$$J45_{pred}\left(\widetilde{M}_{obj}, \widetilde{J0}_{obj}, \widetilde{J45}_{obj}\right) = a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i}$$

ou
modèle 2:

$$M_{pred}\left(\widetilde{M}_{obj}, \widetilde{J0}_{obj}, \widetilde{J45}_{obj}, \widetilde{J0}_{sub}, \widetilde{J45}_{sub}\right)$$
$$= \sum_{i=0}^{4} a_{M,i}^{M} \widetilde{M}_{obj}^{i} + a_{J0,1}^{M} \widetilde{J0}_{obj} + a_{J45,1}^{M} \widetilde{J45}_{obj} + b_{J0,1}^{M} \widetilde{J0}_{sub} + b_{J45,1}^{M} \widetilde{J45}_{sub}$$

$$J0_{pred}\left(\widetilde{M}_{obj}, \widetilde{J0}_{obj}, \widetilde{J45}_{obj}, \widetilde{J0}_{sub}, \widetilde{J45}_{sub}\right)$$
$$= a_{M,1}^{J0} \widetilde{M}_{obj} + \sum_{i=0}^{4} a_{J0,i}^{J0} \widetilde{J0}_{obj}^{i} + a_{J45,1}^{J0} \widetilde{J45}_{obj} + b_{M,1}^{J0} \widetilde{M}_{sub} + b_{J45,1}^{J0} \widetilde{J45}_{sub}$$

$$J45_{pred}\left(\widetilde{M}_{obj}, \widetilde{J0}_{obj}, \widetilde{J45}_{obj}, \widetilde{J0}_{sub}, \widetilde{J45}_{sub}\right)$$
$$= a_{M,1}^{J45} \widetilde{M}_{obj} + a_{J0,1}^{J45} \widetilde{J45}_{obj} + \sum_{i=0}^{4} a_{J45,i}^{J45} \widetilde{J45}_{obj}^{i} + b_{M,1}^{J45} \widetilde{M}_{sub} + b_{J0,1}^{J45} \widetilde{J0}_{sub}$$

où:

($M_{pred}$, $J0_{pred}$, $J45_{pred}$) désigne le vecteur de puissance de la réfraction prédite;($\widetilde{M}_{obj}$, $\widetilde{J0}_{obj}$, $\widetilde{J45}_{obj}$) désigne le vecteur de puissance des valeurs mesurées à partir de la réfraction objective;($\widetilde{M}_{sub}$, $\widetilde{J0}_{sub}$, $\widetilde{J45}_{sub}$) désigne le vecteur de puissance des valeurs de mesure issues de la réfraction subjective;

$a_{X,i}^Y$ et $b_{Z,i}^Y$ désignent les paramètres de la fonction paramétrique correspondante,

Y représente une composante de vecteur de puissance du vecteur de puissance de la réfraction prédite ;

X représente une composante de vecteur de puissance du vecteur de puissance de la réfraction objective mesurée ; et

$\underline{Z}$ représente une composante de vecteur de puissance du vecteur de puissance de la réfraction subjective mesurée.

5. Procédé selon l'une quelconque des revendications précédentes, dans lequel le calcul de l'estimation de l'amétropie de l'œil comprend la formation d'une moyenne pondérée des valeurs mesurées à partir de la première mesure et de la deuxième mesure, la première mesure étant pondérée par des premiers poids et la deuxième mesure étant pondérée par des deuxièmes poids, optionnellement, parmi la première mesure et la deuxième mesure, la mesure ayant la plus faible incertitude de mesure étant pondérée par des poids plus élevés.

6. Procédé selon la revendication 5, si l'on se réfère à la revendication 3, dans lequel les poids dépendent des valeurs de mesure de l'amétropie.

7. Procédé selon la revendication 6, dans lequel les valeurs de mesure comprennent une addition et/ou un équivalent sphérique et les poids dépendent de l'addition et/ou de la différence entre la valeur de mesure de l'équivalent sphérique provenant de la première mesure et la valeur de mesure de l'équivalent sphérique provenant de la deuxième mesure.

8. Procédé selon l'une quelconque des revendications précédentes, dans lequel :

les incertitudes de mesure ou les écarts de mesure de la première mesure et de la deuxième mesure sont déterminés pour la distance de l'objet à l'infini ; et/ou

les incertitudes ou écarts de mesure de la première mesure et de la deuxième mesure sont déterminés séparément pour différents appareils ;

les incertitudes ou écarts de mesure de la première mesure et de la deuxième mesure sont déterminés à une distance de l'œil identique pour toutes les données.

9. Produit programme d'ordinateur qui, lorsqu'il est chargé dans la mémoire d'un ordinateur et exécuté sur celui-ci, amène l'ordinateur à mettre en oeuvre un procédé selon l'une quelconque des revendications 1 à 8.

10. Dispositif pour déterminer le défaut de vision d'un oeil d'un porteur de lunettes, comprenant un dispositif de calcul adapté pour mettre en oeuvre le procédé selon l'une quelconque des revendications 1 à 8.

11. Procédé de fabrication d'un verre de lunettes, comprenant :

déterminer l'amétropie d'un oeil du porteur de lunettes selon le procédé selon l'une des revendications 1 à 8 ;

déterminer de l'action de consigne à partir de l'amétropie déterminée, de sorte que l'action de consigne du verre de lunettes corrige au moins partiellement l'amétropie déterminée en au moins un point de référence ; et

fabriquer le verre de lunettes de telle sorte que l'effet de consigne soit atteint en au moins un point de référence prédéterminé du verre de lunettes.

12. Dispositif pour la fabrication d'un verre de lunettes comprenant ;

un dispositif pour déterminer l'amétropie d'un oeil d'un porteur de lunettes selon la revendication 10 ;

un dispositif pour déterminer l'effet de consigne en un point de référence du verre de lunettes à l'aide de l'amétropie déterminée, et

un dispositif de fabrication pour fabriquer le verre de lunettes de telle sorte que l'effet de consigne soit atteint dans l'au moins un point de référence du verre de lunettes, de préférence dans une position d'utilisation prédéterminée du verre de lunettes.

13. Procédé mis en oeuvre par ordinateur pour commander des verres de lunettes, comprenant :

fournir des valeurs mesurées à partir d'une première mesure et d'une deuxième mesure de l'amétropie de l'œil du porteur de lunettes ;
calculer une estimation de l'amétropie de l'œil du porteur de lunettes selon le procédé de l'une des revendications 1 à 8.

14. Dispositif de commande de verres de lunettes comprenant :

un dispositif pour fournir des valeurs de mesure à partir d'une première mesure et d'une deuxième mesure de l'amétropie de l'œil du porteur de lunettes, et
un dispositif de calcul qui est conçu pour calculer une valeur estimée de l'amétropie de l'œil du porteur de lunettes à l'aide des valeurs de mesure provenant de la première mesure et de la deuxième mesure selon le procédé selon l'une des revendications 1 à 8.

## Systematische Unterschiede M

**Fig. 1A**

## Systematische Unterschiede J0

**Fig. 1B**

Systematische Unterschiede J45

Fig. 1C

## Systematische Unterschiede M

**Fig. 2A**

## Systematische Unterschiede J0

**Fig. 2B**

Fig. 2C

Fig. 3

Fig. 4A

Fig. 4B

Unterschiede Verfahren 1 – Verfahren 2 von M kombiniert Ferne mesopisch, Gerät 1

AVG_NEU_ALT_Kmb_F_mes_M / dpt

## Fig. 5A

Unterschiede Verfahren 1 – Verfahren 2 von J0 kombiniert Ferne mesopisch, Gerät 1

AVG_NEU_ALT_Kmb_F_mes_J0 / dpt

## Fig. 5B

Unterschiede Verfahren 1 – Verfahren 2 von J45 kombiniert Ferne mesopisch, Gerät 1

## Fig. 5C

Unterschiede Verfahren 1 – Verfahren 2 von M kombiniert Ferne mesopisch, Gerät 2

## Fig. 6A

Unterschiede Verfahren 1 – Verfahren 2 von J0 kombiniert Ferne mesopisch, Gerät 2

## Fig. 6B

Unterschiede Verfahren 1 – Verfahren 2 von J45 kombiniert Ferne mesopisch, Gerät 2

**Fig. 6C**

EP 3 776 068 B1

Beispiel:   M_A1 = 4.1 dpt
            M_A2 = 4.6 dpt
            M_A = 4.1 dpt

**Fig. 7**

**Fig. 8**

EP 3 776 068 B1

Fig. 9

Fig. 10

**Add = 0dpt**

Fig. 11

Add = 0.75dpt

Fig. 12

Fig. 13

**Add = 1.25dpt**

Fig. 14

**Add = 1.5dpt**

**Fig. 15**

Fig. 16

**Add = 2dpt**

Fig. 17

Add = 2.25dpt

Fig. 18

**Add = 2.5dpt**

Fig. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009007136 A1
- US 2012069297 A
- US 2014368795 A1

**In der Beschreibung aufgeführte Nicht-Patentliteratur**

- **L. THIBOS et al.** *Journal of Vision,* April 2004, vol. 4, 9
- **ISKANDER et al.** *Ophthal. Physiol. Opt.,* 2007, vol. 27, 245-255
- **L. THIBOS et al.** Power Vectors: An Application of Fourier Analysis to the Description and Statistical Analysis of Refractive Error. *Optometry and Vision Science,* vol. 74 (6), 367-375